(19) Europäisches Patentamt / European Patent Office / Office européen des brevets

(11) **EP 4 557 235 A1**

(12) **EUROPEAN PATENT APPLICATION**
published in accordance with Art. 153(4) EPC

(43) Date of publication:
21.05.2025 Bulletin 2025/21

(51) International Patent Classification (IPC):
*G06V 10/764* (2022.01)

(21) Application number: 23870756.6

(22) Date of filing: 25.09.2023

(86) International application number:
PCT/CN2023/121265

(87) International publication number:
WO 2024/067527 (04.04.2024 Gazette 2024/14)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 27.09.2022 CN 202211180701

(71) Applicant: Wuhan United Imaging Healthcare Co.,
Ltd.
WuHan, Hubei 430206 (CN)

(72) Inventors:
• LI, Chuandong
  Wuhan, Hubei 430206 (CN)
• LIU, Anyi
  Wuhan, Hubei 430206 (CN)
• WAN, Qi
  Wuhan, Hubei 430206 (CN)

(74) Representative: Wang, Bo
Panovision IP
Ebersberger Straße 3
85570 Markt Schwaben (DE)

(54) **HIP JOINT ANGLE MEASUREMENT SYSTEM AND METHOD**

(57) Embodiments of the present disclosure provide a system and method for detecting an angle of a hip joint. The system comprises a processor, and the processor is configured to perform following operations: obtaining an ultrasound image of a subject to be detected; extracting a contour of an ilium region corresponding to a hip joint of the subject to be detected from the ultrasound image; obtaining a position of a centroid of the ilium region in the contour of the ilium region, obtaining an image of the hip joint based on the position of the centroid, and determining an angle of the hip joint of the subject to be detected based on the image of the hip joint.

<u>200</u>

Obtaining an ultrasound image of a subject to be detected — 210

Extracting a contour of an ilium region corresponding to a hip joint of the subject to be detected from the ultrasound image — 220

Obtaining a position of a centroid of the ilium region in the contour of the ilium region and obtaining an image of the hip joint based on the position of the centroid — 230

Determining an angle of the hip joint of the subject to be detected based on the image of the hip joint — 240

**FIG. 2**

EP 4 557 235 A1

**Description**

**CROSS REFERENCE TO RELATED APPLICATIONS**

This specification claims the priority of Chinese Application No. 202211180701.9, filed on September 27, 2022, the entire content of which is incorporated herein by reference.

**TECHNICAL FIELD**

The present disclosure relates to the field of hip joint classification, and in particular, to a system and method for detecting an angle of a hip joint.

**BACKGROUND**

The hip joint classification of a newborn can help prevent or treat hip dysplasia through early screening and intervention, which helps avoid more serious problems of the hip joint in later development, such as hip dislocation, degenerative changes in the hip joint, etc. For newborns, hip joint classification is crucial as it can assist doctors in detecting hip dysplasia early and taking appropriate preventive or therapeutic measures.

Some embodiments of the present disclosure provide a system and a method for detecting an angle of hip joint for better detection of hip joints of newborns.

**SUMMARY**

One or more embodiments of the present disclosure provide a system for detecting an angle of a hip joint. The system comprises a processor, and the processor is configured to obtain an ultrasound image of a subject to be detected; extract a contour of an ilium region corresponding to a hip joint of the subject to be detected from the ultrasound image; obtain a position of a centroid of the ilium region in the contour of the ilium region; obtain an image of the hip joint based on the position of the centroid; and determine an angle of the hip joint of the subject to be detected based on the image of the hip joint.

In some embodiments, the extracting a contour of an ilium region corresponding to a hip joint of the subject to be detected from the ultrasound image includes: filtering the ultrasound image to obtain a filtered ultrasound image; obtaining a binary image including a plurality of contour regions by performing thresholding segmentation on the filtered ultrasound image; and determining the contour of the ilium region based on the binary image.

In some embodiments, a filtering manner includes median filtering, and a thresholding segmentation manner includes maximum entropy thresholding segmentation.

In some embodiments, the determining the contour of the ilium region based on the binary image includes determining the contour of the ilium region by processing the binary image using a preset screening manner.

In some embodiments, the processing the binary image using a preset screening manner includes obtaining one or more candidate connected components from the binary image through a preset screening strategy, the preset screening strategy including using at least one of screening condition in three dimensions of area screening, centroid screening, and length screening; and determining the contour of the ilium region based on the one or more candidate connected components.

In some embodiments, when a count of the one or more candidate connected components is less than 1, the processor is further configured to obtain a downgraded screening strategy by performing dimensionality reduction of screening conditions of the preset screening strategy; and obtain the one or more candidate connected components from the binary image through a downgraded screening strategy.

In some embodiments, an order of the dimensionality reduction on the screening conditions is the length screening, the area screening, and the centroid screening sequentially.

In some embodiments, when the count of the one or more candidate connected components is greater than 1, the processor is further configured to calculate, for each of the one or more candidate connected components, a discrimination score corresponding to the candidate connected component; designate a candidate connected component, among the one or more candidate connected components, with a highest discrimination score as a target connected component; and extract the contour of the ilium region from the ultrasound image based on the target connected component.

In some embodiments, the obtaining an image of the hip joint based on the position of the centroid includes: obtaining an image of the ilium region by expanding in accordance with a preset size based on the position of the centroid of the ilium region in the contour of the ilium region, the preset size correlating with a size of the ultrasound image; and obtaining the image of the hip joint by processing the image of the ilium region using a preset active contour model.

In some embodiments, the determining an angle of the hip joint of the subject to be detected based on the image of the hip joint includes: determining a crest point, an end point, and an acetabular labrum midpoint in the ilium region based on the image of the hip joint; determining a bone apex line by connecting the crest point and the end point; determining a cartilage apex line by connecting the crest point and the acetabular labrum midpoint; and determining the angle of the hip joint of the subject to be detected based on a slope of the bone apex line and a slope of the cartilage apex line.

In some embodiments, the determining the crest point based on the image of the hip joint includes: segmenting the image of the hip joint into a first image and a second image according to a preset baseline; the preset baseline being a straight line formed based on a longitudinal coordinate of a centroid of an ilium in the image of the ilium region; determining an upper borderline of a contour of the ilium in the second image and extracting points on the upper borderline to form a set of points of the bone apex line; obtaining the slope of the bone apex line based on the set of points of the bone apex line; and determining the crest point from the set of points of the bone apex line based on the slope of the bone apex line.

In some embodiments, the determining the end point based on the image of the hip joint includes: designating a point corresponding to a maximum longitudinal coordinate in the set of points of the bone apex line as the end point.

In some embodiments, the determining the acetabular labrum midpoint based on the image of the hip joint includes: segmenting an acetabular labrum region from the first image; and designating a centroid of the acetabular labrum region as the acetabular labrum midpoint.

One or more embodiments of the present disclosure provide a method for detecting an angle of a hip joint. The method comprises a processor and the processor is configured to obtain an ultrasound image of a subject to be detected; extract a contour of an ilium region corresponding to a hip joint of the subject to be detected from the ultrasound image; obtain a position of a centroid of the ilium region in the contour of the ilium region; obtain an image of the hip joint based on the position of the centroid; and determine an angle of the hip joint of the subject to be detected based on the image of the hip joint.

In some embodiments, the extracting a contour of an ilium region corresponding to a hip joint of the subject to be detected from the ultrasound image includes: filtering the ultrasound image to obtain a filtered ultrasound image; obtaining a binary image including a plurality of contour regions by performing thresholding segmentation on the filtered ultrasound image; and determining the contour of the ilium region based on the binary image.

In some embodiments, a filtering manner includes median filtering, and a thresholding segmentation manner includes maximum entropy thresholding segmentation.

In some embodiments, the determining the contour of the ilium region based on the binary image includes determining the contour of the ilium region by processing the binary image using a preset screening manner.

In some embodiments, the processing the binary image using a preset screening manner includes: obtaining one or more candidate connected components from the binary image through a preset screening strategy, the preset filtering strategy including using at least one of screening condition in three dimensions of area screening, centroid screening, and length screening; and determining the contour of the ilium region based on the one or more candidate connected components.

In some embodiments, when a count of the one or more candidate connected components is less than 1, the processor is further configured to: obtain a downgraded screening strategy by performing dimensionality reduction on the screening conditions of the preset screening manner; and obtain the one or more candidate connected components from the binary image through the downgraded screening strategy.

In some embodiments, an order of the dimensionality reduction on the screening conditions is the length screening, the area screening, and the centroid screening sequentially.

In some embodiments, when the count of the one or more candidate connected components is greater than 1, the processor is further configured to: calculate, for each of the one or more candidate connected components, a discrimination score corresponding to the candidate connected component; designate a candidate connected component, among the one or more candidate connected components, with a highest discrimination score as a target connected component; and extract the contour of the ilium region from the ultrasound image based on the target connected component.

In some embodiments, the obtaining an image of the hip joint based on the position of the centroid includes: obtaining an image of the ilium region by expanding in accordance with a preset size based on the position of the centroid of the ilium region in the contour of the ilium region, the preset size correlating with a size of the ultrasound image; and obtaining the image of the hip joint by processing the image of the ilium region using a preset active contour model.

In some embodiments, the determining an angle of the hip joint of the subject to be detected based on the image of the hip joint includes: determining a crest point, an end point, and an acetabular labrum midpoint in the ilium region based on the image of the hip joint; determining a bone apex line by connecting the crest point and the end point; determining a cartilage apex line by connecting the crest point and the acetabular labrum midpoint; and determining the angle of the hip joint of the subject to be detected based on a slope of the bone apex line and a slope of the cartilage apex line.

In some embodiments, the determining the crest point based on the image of the hip joint includes: segmenting the image of the hip joint into a first image and a second image according to a preset baseline; the preset baseline being a straight line formed based on a longitudinal coordinate of a centroid of an ilium in the image of the ilium region; determining an upper borderline of a contour of the ilium in the second image and extracting points on the upper borderline to form a set of points of the bone apex line; obtaining the slope of the bone apex line based on the set of points of the bone apex line; and determining the crest point from the set of points of the bone apex line based on the slope of the bone apex line.

In some embodiments, the determining the end point based on the image of the hip joint includes: designating a point corresponding to a maximum longitudinal coordinate in the set of points of the bone apex line as the end point.

In some embodiments, the determining the acetabular labrum midpoint based on the image of the hip joint includes: segmenting an acetabular labrum region from the first image; and designating a centroid of the acetabular labrum region as the acetabular labrum midpoint.

One or more embodiments of the present disclosure provide a method for classifying a hip joint. The method comprises obtaining an original ultrasound image of a subject to be detected; the original ultrasound image being an ultrasound image corresponding to a hip joint of the subject to be detected; extracting an image of an ilium region corresponding to the hip joint of the subject to be detected from the original ultrasound image; and obtaining a type of the hip joint of the subject to be detected by inputting the image of the ilium region into a preset active contour model for hip joint classification.

In some embodiments, the extracting an image of an ilium region corresponding to the hip joint of the subject to be detected from the original ultrasound image includes: filtering the original ultrasound image to obtain a filtered original image; obtaining a binary image including a plurality of contour regions by performing thresholding segmentation on the filtered original ultrasound image; determining a contour of the ilium region based on the binary image; and determining the image of the ilium region based on the contour of the ilium region.

In some embodiments, the determining the contour of the ilium region based on the binary image includes determining the contour of the ilium region by processing the binary image through a preset screening manner.

In some embodiments, the processing the binary image through a preset screening manner includes obtaining one or more candidate connected components from the binary image through a preset screening strategy, the preset screening strategy including using at least one of screening condition in three dimensions of area screening, centroid screening, and length screening; and determining the contour of the ilium region based on the one or more candidate connected components.

In some embodiments, the determining the image of the ilium region based on the contour of the ilium region includes: obtaining a position of a centroid of the ilium region in the contour of the ilium region; and obtaining the image of the ilium region by expanding in accordance with a preset size based on the position of the centroid of the ilium region in the contour of the ilium region, wherein the preset size correlates with a size of the ultrasound image.

In some embodiments, the obtaining the type of the hip joint of the subject to be detected by inputting the image of the ilium region into a preset active contour model for classification of a hip joint includes: obtaining the image of the hip joint by processing the image of the ilium region using the presser active contour mode; determining a crest point, an end point, and an acetabular labrum midpoint in the ilium region based on the image of the hip joint; determining a bone apex line by connecting the crest point and the end point, and determining a cartilage apex line by connecting the crest point and the acetabular labrum midpoint; determining the angle of the hip joint of the subject to be detected based on a slope of the bone apex line and a slope of the cartilage apex line; and determining the type of the hip joint of the subject to be detected based on the angle of the hip joint of the subject to be detected and a hip joint classification standard.

One or more embodiments of the present disclosure provide a device for classifying a hip joint. The device comprises a processor and the processor is configured to execute a method for classifying a hip joint.

## BRIEF DESCRIPTION OF THE DRAWINGS

The present disclosure will be further illustrated by way of exemplary embodiments, which will be described in detail by means of the accompanying drawings. These embodiments are not limiting, and in these embodiments, the same numbering denotes the same structure, where:

FIG. 1 is a schematic diagram illustrating an exemplary application scenario of a system for classifying a hip joint according to some embodiments of the present disclosure;
FIG. 2 is a flowchart illustrating a process for detecting an angle of a hip joint according to some embodiments of the present disclosure;
FIG. 3 is a flowchart illustrating a process for determining a contour of an ilium region according to some embodiments of the present disclosure;
FIG. 4 is a flowchart illustrating a process for determining an angle of a hip joint according to some embodiments of the present disclosure;
FIG. 5 is a flowchart illustrating a process for classifying a hip joint according to some embodiments of the present disclosure;
FIG. 6 is a flowchart illustrating a process for determining an image of an ilium region according to other embodiments of the present disclosure;

FIG. 7 is a flowchart illustrating a process for performing filtering and thresholding segmentation on an ultrasound image according to some embodiments of the present disclosure;

FIG. 8 is a flowchart illustrating a process for obtaining one or more candidate connected components according to some embodiments of the present disclosure;

FIG. 9 is a flowchart illustrating a process for obtaining a target connected component according to some embodiments of the present disclosure;

FIG. 10 is a flowchart illustrating a process for determining an image of an ilium region according to some embodiments of the present disclosure;

FIG. 11 is a flowchart illustrating a process for classifying a hip joint according to some embodiments of the present disclosure;

FIG. 12 is a flowchart illustrating a process for determining three anatomical points according to some embodiments of the present disclosure;

FIG. 13 is a flowchart illustrating a process for determining a crest point according to some embodiments of the present disclosure;

FIG. 14 is a schematic diagram illustrating an exemplary structure of a system for classifying a hip joint according to some embodiments of the present disclosure;

FIG. 15 is a schematic diagram illustrating an exemplary structure of a method for classifying a hip joint according to some embodiments of the present disclosure;

FIG. 16 is a diagram illustrating an ultrasound image of a subject to be detected according to some embodiments of the present disclosure;

FIG. 17 is a diagram illustrating a binarized segmentation image corresponding to an ultrasound image according to some embodiments of the present disclosure;

FIG. 18 is a diagram illustrating an image of a target connected component corresponding to an ultrasound image according to some embodiments of the present disclosure;

FIG. 19 is a schematic diagram illustrating a process for screening a connected component according to some embodiments of the present disclosure;

FIG. 20 is a diagram illustrating a contour of an ilium region in an ultrasound image according to some embodiments of the present disclosure;

FIG. 21 is a diagram illustrating an image of a hip joint corresponding to a contour of an ilium region according to some embodiments of the present disclosure;

FIG. 22 is a diagram illustrating an image of a hip joint including only a contour of a hip joint according to some embodiments of the present disclosure;

FIG. 23 is a diagram illustrating a classification effect of a hip joint corresponding to an ultrasound image according to some embodiments of the present disclosure; and

FIG. 24 is a schematic diagram illustrating an exemplary internal structure of a processing device according to some embodiments of the present disclosure.

## DETAILED DESCRIPTION

In order to more clearly illustrate the technical solutions of the embodiments of the present disclosure, the accompanying drawings required to be used in the description of the embodiments are briefly described below. Obviously, the accompanying drawings in the following description are only some examples or embodiments of the present disclosure, and it is possible for a person of ordinary skill in the art to apply the present disclosure to other similar scenarios in accordance with these drawings without creative labor. Unless obviously obtained from the context or the context illustrates otherwise, the same numeral in the drawings refers to the same structure or operation.

It should be understood that the terms "system", "device", "unit" and/or "module" as used herein is a way to distinguish between different components, elements, parts, sections, or assemblies at different levels. However, the words may be replaced by other expressions if other words accomplish the same purpose.

As shown in the present disclosure and in the claims, unless the context clearly suggests an exception, the words "a", "an", "one" and/or "the" do not refer specifically to the singular but may also include the plural. Generally, the terms "including" and "comprising" suggest only the inclusion of clearly identified steps and elements that do not constitute an exclusive list, and the method or apparatus may also include other steps or elements.

Flowcharts are used in the present disclosure to illustrate operations performed by a system according to embodiments of the present disclosure. It should be appreciated that the preceding or following operations are not necessarily performed in an exact sequence. Instead, steps can be processed in reverse order or simultaneously. Also, it is possible to add other operations to these processes or remove a step or steps from them.

Currently, for common hip joint diseases in newborns, ultrasound detection combined with the Graf method is typically used to measure the $\alpha$ angle and the $\beta$ angle of the newborn's hip joint. Based on the measured $\alpha$ and $\beta$ angles, the

developmental type of the newborn's hip joint is determined, such as whether it is normally developed or developed abnormally.

In some embodiments, the measurement of the angle of the hip joint of a newborn is carried out by using a manual measurement manner, an automated measurement manner, etc.

The manual measurement manner usually requires the doctor to manually select five anatomical points of the hip joint based on an ultrasound image of the hip joint of the newborn, and use the five anatomical points to automatically calculate the bone apex angle ($\alpha$ angle) and cartilage apex angle ($\beta$ angle) of the hip joint. The five anatomical points are: the upper edge of the reflected head of the rectus femoris and the transition area of the ilium periosteum, the lower edge of the reflected head of the rectus femoris and the transition area of the ilium periosteum, the bone margin turning point, the lowest point of the ilium branch, and the acetabular labrum midpoint. The manual measurement manner is relatively subjective, and the accuracy heavily depends on the doctor's experience. Additionally, the doctor has to perform frequent operations, leading to low work efficiency.

The automated measurement manner may be performed based on hip joint classification or an deep learning. The automated measurement manner based on hip joint classification includes using a method for classifying a hip joint based on a region-based active contour model o, and the method includes after performing preliminary processing on an ultrasound image of a hip joint of a newborn, performing image segmentation using the region-based active contour model to obtain a tissue contour of the hip joint, and then using linear fitting to obtain a bone apex angle and a cartilage apex angle, which directly uses a preliminary pre-processed ultrasound image and input the image to the region-based active contour model, which has a large impact on both speed and accuracy. The automated measurement manner based on hip joint classification includes manually obtaining a region of interest after performing mean filtering on an input ultrasound image, and then combining image enhancement as well as a binarization strategy, which are in turn combined with a linear fitting to obtain a bone apex angle and a cartilage apex angle, which is a semi-automatic measurement manner and relies on the manual acquisition of the region of interest and increases the workload.

The automated measurement manner based on deep learning is to obtain a position and a target measurement value of a target key point of a hip joint of a new born, and input the position and the target measurement value into a deep learning network for training to obtain a network model, which realizes functions of inputting an ultrasound image and outputting a target position. However, the method relies on huge ultrasound image data, but often involves issues such as patient privacy and small amounts of data, which makes it difficult to be executed or to render a more obvious effect based on a small amount of data.

Aiming at the technical problems existing in the traditional method of measuring an angle of a hip joint of a newborn, some embodiments of the present disclosure propose a system and method for detecting an angle of a hip joint that can improve the processing efficiency of hip joint classification, and also ensure that a classification result is objective and accurate.

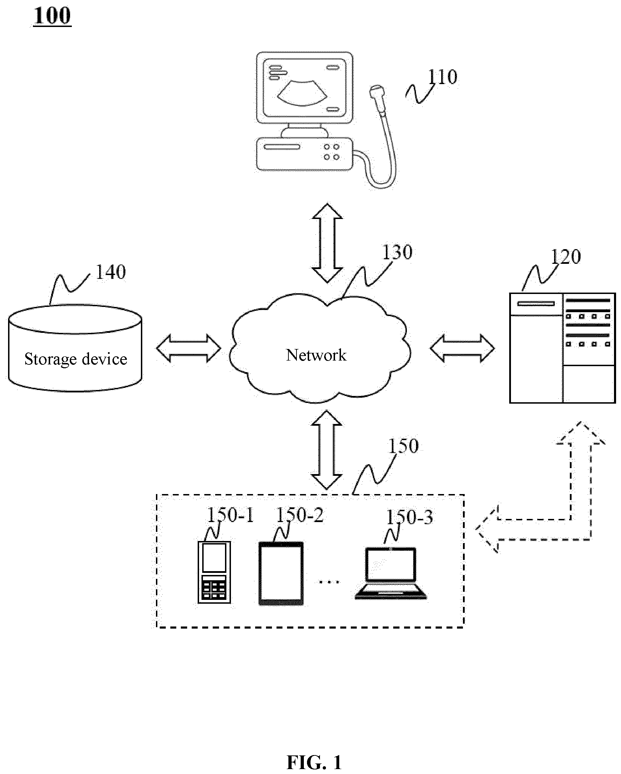

FIG. 1 is a schematic diagram illustrating an exemplary application scenario of a system for detecting an angle of a hip joint according to some embodiments of the present disclosure.

As shown in FIG. 1, a system for detecting an angle of a hip joint 100 includes an ultrasound imaging device 110, a processing device 120, a network 130, a storage device 140, and a terminal 150. In some embodiments, the system for detecting an angle of a hip joint 100 is configured to detect an angle of a hip joint and classify a hip joint.

The ultrasound imaging device 110 is configured to obtain ultrasound imaging data of a target region of a subject to be detected. In some embodiments, an ultrasound imaging device is configured to obtain ultrasound imaging data of a target region of a subject by utilizing physical properties of ultrasound waves and a difference in acoustic properties of the target region of the subject. The ultrasound imaging data may be displayed and/or recorded as waveforms, curves, or images of features associated with the target region of the subject. By way of example only, the ultrasound imaging device includes one or more ultrasound probes for emitting ultrasound waves to the target region (e.g., a subject located on a treatment bed or an organ, or tissue thereof). Ultrasound waves that pass-through organs and tissues with different acoustic impedances and different attenuation characteristics produce different reflections and attenuation, resulting in echoes that can be received by one or more ultrasound probes. The ultrasound imaging device may process (e.g., amplify, convert) and/or display received echoes to generate the ultrasound imaging data. In some embodiments, the ultrasound imaging device includes a B-ultrasound device, a color Doppler ultrasound device, a cardiac ultrasound device, a 3D color ultrasound device, etc., or any combination thereof.

In some embodiments, the ultrasound imaging device 110 is configured to send the ultrasound imaging data via the network 130 to the processing device 120, the storage device 140, and/or the terminal 150 for further processing. For example, the ultrasound imaging data obtained by the ultrasound imaging device is non-image data, and the non-image data is sent to the processing device 120 for generating an ultrasound image. As another example, the ultrasound imaging data obtained by the ultrasound imaging device may be image data, and the image data is sent to the terminal 150 for display. As another example, the ultrasound imaging data is stored in the storage device 140.

The processing device 120 is configured to process data and/or information obtained from the ultrasound imaging device 110, the storage device 140, and/or the terminal 150. For example, the processing device 120 is configured to process ultrasound imaging data obtained from an imaging device in the ultrasound imaging device 110 and generate an ultrasound image of a target region. In some embodiments, the ultrasound image is sent to the terminal 150 and displayed on one or more display devices in the terminal 150. In some embodiments, the processing device 120 is a single server or group of servers. The group of servers may be centralized or distributed. In some embodiments, the processing device 120 is local or remote. For example, the processing device 120 is configured to access information and/or data stored in the ultrasound imaging device 110, the storage device 140, and/or the terminal 150 via the network 130. As another example, the processing device 120 is directly connected to the ultrasound imaging device 110, the storage device 140, and/or the terminal 150 to access information and/or data stored thereon. As another example, the processing device 120 is integrated in the ultrasound imaging device 110. In some embodiments, the processing device 120 is implemented on a cloud platform. By way of example only, the cloud platform includes a private cloud, a public cloud, a hybrid cloud, a community cloud, a distributed cloud, an on-premises cloud, a multi-cloud, etc., or any combination thereof.

In some embodiments, the processing device 120 is a single processing device in communication with the ultrasound imaging device and is configured to process data received from the ultrasound imaging device.

The network 130 may include any suitable network that may facilitate the exchange of information and/or data for the system for detecting an angle of a hip joint 100. In some embodiments, one or more components of the system for detecting an angle of a hip joint 100 (e.g., the ultrasound imaging device 110, the processing device 120, the storage device 140, or the terminal 150) are connected and/or in communication with other components of the system for detecting an angle of a hip joint 100 via the network 130. For example, the processing device 120 is configured to obtain the ultrasound imaging data from the ultrasound imaging device 110 via the network 130. As another example, the processing device 120 is configured to obtain a user instruction from the terminal 150 via the network 130. The instruction is configured to instruct the ultrasound imaging device 110 to perform imaging. In some embodiments, the network 130 includes one or more network access points. For example, the network 130 includes wired and/or wireless network access points, such as a base station and/or an Internet access point, through which one or more of the components of the system for detecting an angle of a hip joint 100 may connect to the network 130 to exchange data and/or information.

The storage device 140 may store data and/or instructions. In some embodiments, the storage device 140 is configured to store data obtained from the terminal 150 and/or the processing device 120. In some embodiments, the storage device 140 is configured to store data and/or instructions that the processing device 120 can execute or use to execute exemplary methods described in the present disclosure. In some embodiments, the storage device 140 is implemented on a cloud platform. By way of example only, the cloud platform includes a private cloud, a public cloud, a hybrid cloud, a community cloud, a distributed cloud, an on-premises cloud, a multi-cloud, etc., or any combination thereof.

In some embodiments, the storage device 140 is connected to the network 130 to communicate with one or more components of the system for detecting an angle of a hip joint 100 (e.g., the processing device 120, the terminal 150, etc.). One or more components of the system for detecting an angle of a hip joint 100 may access data or instructions stored in the storage device 140 via the network 130. In some embodiments, the storage device 140 is directly connected to or in communication with one or more components of the system for detecting an angle of a hip joint 100 (e.g., the processing device 120, the terminal 150, etc.). In some embodiments, the storage device 140 is part of the processing device 120.

The terminal 150 may include a mobile device 150-1, a tablet 150-2, a laptop 150-3, etc., or any combination thereof. In some embodiments, the terminal 150 may remotely operate the ultrasound imaging device 110. In some embodiments, the terminal 150 may operate the ultrasound imaging device 110 via a wireless connection. In some embodiments, the terminal 150 may receive information and/or instructions entered by a user and send received information and/or instructions to the ultrasound imaging device 110 or the processing device 120 via the network 130. In some embodiments, the terminal 150 may receive data and/or information from the processing device 120. In some embodiments, the terminal 150 may be part of the processing device 120. In some embodiments, the terminal 150 may be omitted.

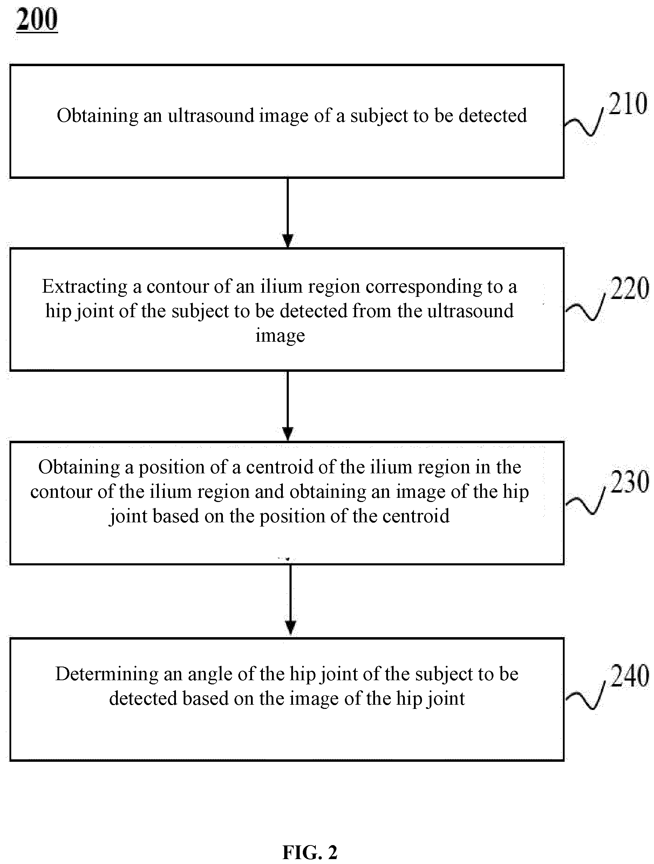

FIG. 2 is a flowchart illustrating a process for detecting an angle of a hip joint according to some embodiments of the present disclosure. In some embodiments, a process 200 may be performed by a processing device (e.g., the processing device 120) or a system for detecting an angle of a hip joint (e.g., the system for detecting an angle of a hip joint 100). For example, the process 200 may be stored in a storage device (e.g., a self-contained storage unit of a processing device or an external storage device) in the form of a program or an instruction, and the program or instruction realizes the process 200 when executed. The process 200 may include following operations.

In step 210, an ultrasound image of a subject to be detected is obtained.

The subject to be detected may include patients or other medical subjects (e.g., test models, etc.). The subject to be detected may also be part of a patient (e.g., a newborn) or other medical laboratory subject, including organs and/or tissues, e.g., an iliac bone, etc.

The ultrasound image may be an image obtained after detecting the subject to be detected using an ultrasound device. An ultrasound signal is typically generated by high-frequency sound waves emitted by an ultrasound probe, which are reflected from different types of tissues or organs encountered within the subject to be detected. These reflected sound waves are received by the ultrasound probe, after amplification, filtering, digitization, and other processing, are ultimately converted into an image, which is the ultrasound image. In some other places in the present disclosure, the ultrasound image obtained is also referred to as an original ultrasound image.

In step 220, a contour of an ilium region corresponding to a hip joint of the subject to be detected is extracted from the ultrasound image.

The ilium is one of the components of a hip bone, forming the posterior upper part of the hip bone. The ilium includes an ilium body and an ilium wing. The ilium region refers to a region located on the lateral side of the torso, above the hip bone. The range of the ilium region roughly corresponds to a region between the waist and the hips. The ilium is one of the largest flat bones in the human body. The iliac crest on the ilium is one of the primary origin points for the femur muscles. The acetabulum of the ilium and the caput femoris form the hip joint.

The hip joint is one of the largest joints in the human body, connecting the hip bone to the femur. The hip joint is a ball-and-socket joint, consisting of the caput femoris, the acetabulum, the acetabular labrum, etc. The hip joint has a very important role in human movement, supporting the body's weight and walking, running, and other activities. At the same time, the hip joint plays a key role in body posture and balance control.

The contour of the ilium region is an image including a contour of an ilium that can be used to locate and identify the ilium and its associated structures. For example, the contour of the ilium region includes a contour of a structure such as the ilium.

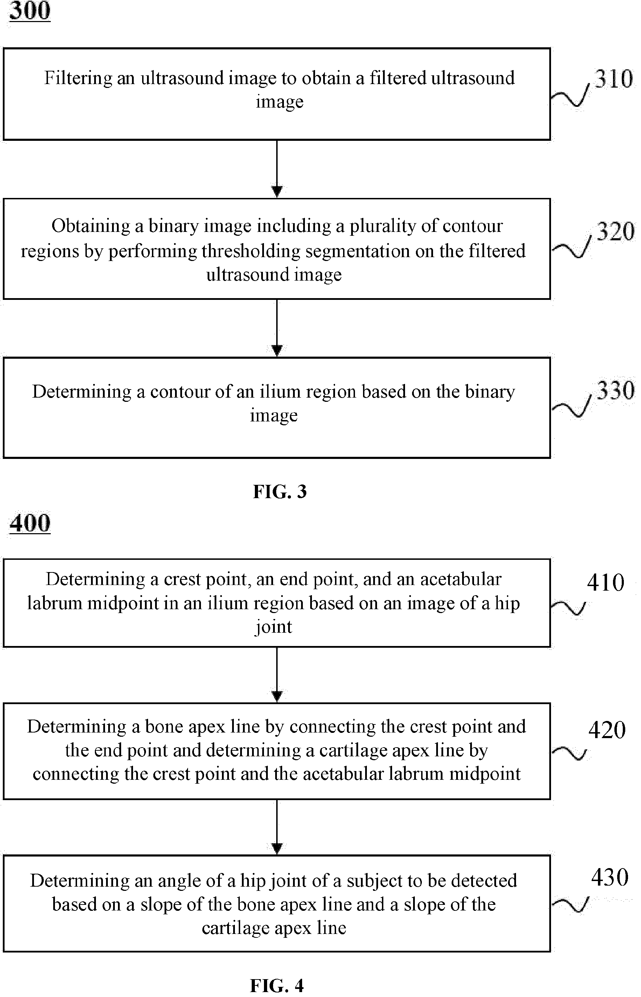

In some embodiments, the processing device may also obtain the contour of the ilium region using methods such as target detection, key point detection, and recognition of image features. In some embodiments, the processing device may also filter the ultrasound image to obtain a filtered ultrasound image, perform thresholding segmentation on the filtered ultrasound image, and obtain the contour of the ilium region based on a result of the thresholding segmentation. A detailed description thereof can be found in FIG. 3 and the related descriptions thereof.

In step 230, a position of a centroid of the ilium region in the contour of the ilium region is obtained, and an image of the hip joint based on the position of the centroid is obtained.

The centroid refers to an average position of all mass points inside a subject. The position of the centroid of the ilium region refers to an average position of all the elemental points in a region defined by the contour of the ilium region in the ultrasound image.

In some embodiments, the processing device may obtain the position of the centroid of the contour of the ilium region in various ways, e.g., a method based on mathematical calculations, a method based on deep learning, etc., which is not limited in the present disclosure. For example, the processing device may obtain the position of the centroid of the ilium region in a following manner.

The processing device may extract an image of the ilium region from the ultrasound image based on the position of the centroid of the ilium region in the contour of the ilium region and obtain an image of the hip joint based on the image of the ilium region. The image of the hip joint is an image representing a structure of the hip joint of the subject to be detected segmented from the image of the ilium region. Thus, in some embodiments, the image of the hip joint is also referred to as a segmentation image of the hip joint.

In some embodiments, the processing device may obtain the image of the hip joint based on the position of the centroid of the ilium region using a preset active contour model. For example, the processing device may obtain the image of the ilium region by expanding the position of the centroid of the ilium region in the contour of the ilium region in accordance with a preset size. The processing device may obtain the image of the hip joint by processing the image of the ilium region using the preset active contour model. More descriptions about obtaining the image of the ilium region can be found in the description of step 502.

The image of the ilium region is a portion of the ultrasound image that represents the ilium.

Expansion refers to that expansion from the position of the centroid in all directions (e.g., up, down, left, right, and four directions). It can be understood that the position of the centroid is located within the ilium region, and expanding from the position of the centroid in all directions can obtain a more accurate and complete image of the ilium region to minimize other background regions in the image of the ilium region.

The preset size refers to a preset expanded size/length. The preset size may include a preset length and a preset width, and the preset length and the preset width may be expressed in terms of a pixel distance, e.g., a 100-pixel distance, a 200-pixel distance, and so on. In some embodiments, the preset size is related to the size of the ultrasound image. For example, the preset size may be a certain proportion of the size of the ultrasound image, e.g., if the ultrasound image is 1000*200 pixels in size, the preset size may be 500 and 100. As another example, the preset size may be the size of the ultrasound image minus a certain size. As another example, if more longitudinal image information is needed when segmenting the hip joint, an image with half the width and four-fifths of the height of an original image may be obtained.

In this embodiment, expanding the position of the centroid in accordance with the preset size, with the preset size correlating to the ultrasound image, ensures that the hip joint can be completely included in the image of the ilium region obtained based on the expansion.

The preset active contour model may be an image segmentation method based on energy minimization. It can adaptively adjust the shape and position in the image to achieve effective extraction of the hip joint.

In some embodiments, the processing device may input the image of the ilium region to the preset active contour model for processing, and output the image of the hip joint from the preset active contour model.

In some embodiments, the processing device may also process the image of the ilium region in other ways, such as obtaining the image of the hip joint by image segmentation, key point detection, or the like.

In some embodiments, the preset active contour model (also referred to as an active contour model) may also be a machine learning model obtained by training based on sample images of an ilium region and corresponding gold-standard images of a hip joint each of which corresponds to one of the sample images of the ilium region. A training method may include a model training method, e.g., a gradient descent algorithm, etc., which is not limited in this embodiment. In some embodiments, the preset active contour model may also be obtained based on the sample images of the ilium region and gold-standards corresponding to the sample images of the ilium region. Thus, in some embodiments, the processing device may also directly input the image of the ilium region to the active contour model to obtain the segmentation image of the hip joint. Exemplarily, the following is an example of the image of the ilium region being input to the active contour model for processing.

The active contour model may segment the hip joint in the image of the ilium region in an iterative manner, which may include a variety of segmentation methods such as Snake segmentation model, a level set segmentation manner, etc. Exemplarily, the level set segmentation manner is used in embodiments of the present disclosure. The level set manner is a digital method for tracking contours and surface motion. It does not directly operate on the contour but instead converts the contour into the zero-level set of a high-dimensional function, which is also referred to as a level set function. The level set function is then differentiated to obtain an output, and by extracting the zero-level set from the output, the moving contour is obtained. Assuming the input image is $u_0$, and an ilium region curve is denoted as C, the input image is divided into two portions: inside and outside through the curve C. An energy function related to the curve C is represented as:

$$\mathrm{F}(C) = \mu \cdot Length(C) + v \cdot Area\big(inside(C)\big) + \lambda_1 \int_{inside(C)} |u_0(x,y) - c1|^2 dxdy$$

$$+\lambda_2 \int_{outside(C)} |u_0(x,y) - c2|^2 dxdy \qquad (1)$$

where $\mu$, $v$, $\lambda_1$, $\lambda_2$ denote weights, constant c1 denotes a mean value of pixel points inside the curve C, constant c2 denotes a mean value of pixel points outside the curve C, Length(C) denotes a length of the curve C, Area(inside(C)) denotes an area inside the curve C, inside(C) denotes an interior of the curve C, outside(C) denotes an outside of the curve C, and $u_0$ (x, y) denotes a pixel point of the input image $u_0$. The level set manner may be used to obtain the ilium region by minimizing the energy function in an iterative manner, which in turn enables segmentation.

Equation (1) may be converted into an energy function with respect to a level set function $\phi$ expressed as:

$$F_\varepsilon(c1, c2, \phi) = \mu \int_\Omega \delta_\varepsilon\big(\phi(x,y)\big)|\nabla\phi(x,y)|dxdy + v \int_\Omega H_\varepsilon\big(\phi(x,y)\big)|\nabla\phi(x,y)|dxdy$$

$$+\lambda_1 \int_\Omega |u_0(x,y) - c1|^2 H_\varepsilon\big(\phi(x,y)\big)dxdy$$

$$+\lambda_2 \int_\Omega |u_0(x,y) - c2|^2 \Big(1 - H_\varepsilon\big(\phi(x,y)\big)\Big) dxdy \qquad (2)$$

Where the Heaviside function $H_\varepsilon(\phi(x,y))$ and the Delta function $\delta_\varepsilon(\phi(x,y))$ are denoted as following:

$$H_\varepsilon(z) = \frac{1}{2}\Big(1 + \frac{2}{\pi}\arctan\big(\tfrac{z}{\varepsilon}\big)\Big) \qquad (3)$$

$$\delta_\varepsilon(z) = \frac{d}{dz}H(z) = \frac{\varepsilon}{\pi} \cdot \frac{1}{z^2+\varepsilon^2} \qquad (4)$$

Where z denotes $\phi(x, y)$ and c1 and c2 may be determined by $\phi$ as follows:

$$\begin{cases} c_1(\phi) = average(u_0) \ in \ \{\phi \geq 0\} \\ c_2(\phi) = average(u_0) \ in \ \{\phi < 0\} \end{cases} \qquad (5)$$

If c1 and c2 remain unchanged and $F_\varepsilon$ is minimized with respect to $\phi$, the Euler-Lagrange formula is obtained by the variational method as follows:

$$\frac{\partial \phi}{\partial t} = \delta_\varepsilon(\phi)\left[\mu\, div\left(\frac{\nabla \phi}{|\nabla \phi|}\right) - v - \lambda_1(u_0 - c1)^2 + \lambda_2(u_0 - c2)^2\right] = 0 \text{ in } (0,\infty) \times \Omega,$$

$$\phi(0,x,y) = \phi_0(x,y)\ in\ \Omega,$$

$$\frac{\delta_\varepsilon(\phi)}{|\nabla \phi|}\frac{\partial \phi}{\partial \vec{n}} = 0\ on\ \partial \Omega \qquad\qquad (6)$$

It can be seen that steps of the level set manner mainly contain following steps: (1) initializing $\phi^0 = \phi_0$ and $n = 0$; (2) computing the mean values c1 and c2 according to Equation (5); (3) solving $\phi^{n+1}$ according to the partial differential equations of Equation (6); (4) re-initializing $\phi$ using $\phi^{n+1}$; and (5) determining whether a convergence occurs, and if the convergence occurs, then stopping the steps of the level set manner, otherwise, repeating step (2).

The level set manner is used to determine the minimum value of the energy function by iterations to achieve the goal of curve evolution, until a set count of iterations is reached, and the iterations is terminated.

In step 240, an angle of the hip joint of the subject to be detected is determined based on the image of the hip joint.

The angle of the hip joint is an index used to assess an angle between the neck portion of the caput femoris and an axis of the shaft of femur. The angle of the hip joint includes the alpha angle and the beta angle, which can be used for hip joint classification of newborns. In some embodiments, the alpha angle is also referred to as a bone apex angle and the beta angle is also referred to as a cartilage apex angle.

In some embodiments, the processing device may determine the alpha angle and the beta angle by marking out a bone apex line and a cartilage apex line in the image of the hip joint, and then determine the alpha angle and the beta angle by measuring or determining slopes of the bone apex line and the cartilage apex line.

More descriptions about determining the angle of the hip joint can be found in FIG. 4 and the related descriptions thereof.

In some embodiments of the present disclosure, by extracting the ilium region of the ultrasound image, and by segmenting the image based on the position of the centroid of the ilium region, the image of the hip joint of the subject to be detected can be accurately obtained. In turn, based on the image of the hip joint, the angle of the hip joint of the subject to be detected can be determined. Based on a preset classification template of a hip joint (classification condition of a hip joint), a type of the hip joint of the subject to be detected can be accurately determined. A segmentation method of a hip joint based on the position of the centroid can accurately segment a region of the hip joint and shorten the computation time, improve a segmentation rate and segmentation accuracy, and thus achieve the goal of improving the accuracy and processing efficiency of hip joint classification.

FIG. 3 is a flowchart illustrating a process for determining a contour of an ilium region according to some embodiments of the present disclosure. In some embodiments, a process 300 may be performed by a processing device (e.g., the processing device 120).

In step 310, an ultrasound image is filtered to obtain a filtered ultrasound image.

Filtering is a signal processing technique that can be used to change the frequency feature of a signal or to reduce noise. Filtering can be categorized into different types, such as low-pass filtering, high-pass filtering, bandpass filtering, band-stop filtering, etc.

In some embodiments, the processing device may filter the ultrasound image by a variety of filtering methods, for example, mean filtering, Gaussian filtering, median filtering, edge-preserving filtering, or the like.

In step 320, a binary image including a plurality of contour regions is obtained by performing thresholding segmentation on the filtered ultrasound image.

The binary image is an image that contains only two colors, for example, black and white. Each pixel in the binary image includes one of these two colors, with black representing a 0 or low-intensity value and white representing a 1 or high-intensity value.

The plurality of contour regions may be contouring structures of tissues or organs in a segmented ultrasound image. A contour region is usually a region in the ultrasound image where a set of pixel points in the ultrasound image are connected, e.g., a connected component.

The thresholding segmentation is a binarization method based on gray values of an image. The thresholding segmentation can be used to divide tissue structures or regions of different shades of gray in the ultrasound image into two parts: foreground (signal) and background (noise). The basic principle is to set a suitable threshold, then set pixel points in the ultrasound image whose gray values are smaller than the threshold as the background, and pixel points whose gray values are larger than the threshold as the foreground.

In some embodiments, the processing device may process the filtered ultrasound image to obtain the binary image using a preset thresholding segmentation algorithm. The preset thresholding segmentation algorithm may include a maximum entropy thresholding segmentation algorithm, an Otsu thresholding segmentation algorithm, an adaptive thresholding segmentation algorithm, a fixed thresholding segmentation algorithm, etc.

In some embodiments, the processing device may obtain a better processing result by filtering the ultrasound image using the median filtering and segmenting the filtered ultrasound image using the maximum entropy thresholding segmentation algorithm. The median filtering and the maximum entropy thresholding segmentation algorithm have high suitability, and a combination of the median filtering and the maximum entropy thresholding segmentation algorithm can make the segmentation of the ilium region more effective. It should be understood that the processing device may also use other combinations, such as the median filtering and the Otsu thresholding segmentation, which is not limited in the present disclosure.

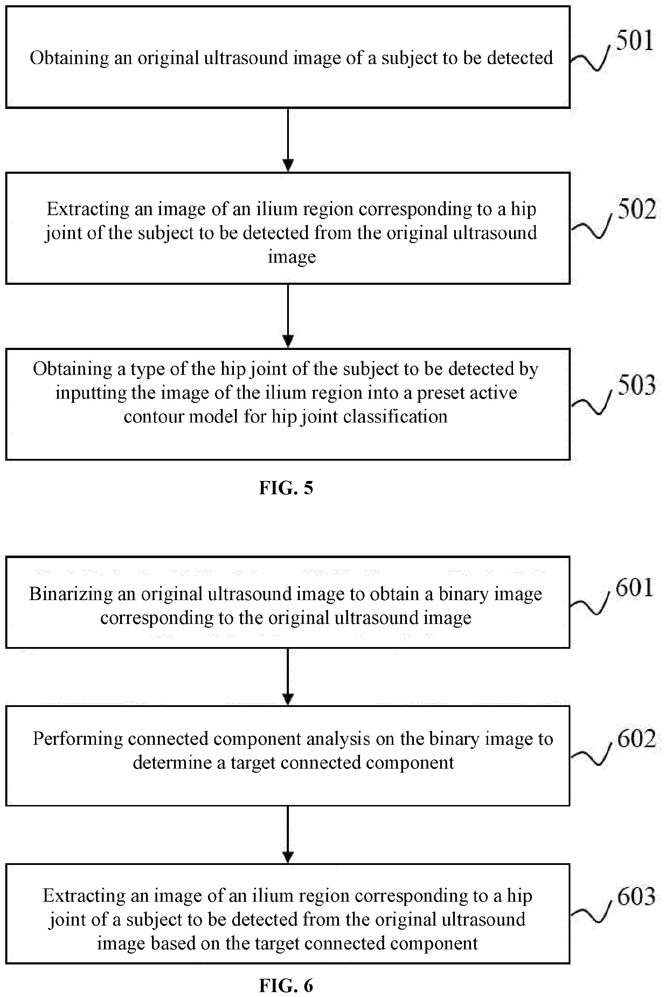

In some other places in the present disclosure, a process of filtering the ultrasound image as well as the thresholding segmentation is also called a binarization processing, and more descriptions can be found in the related instructions of FIG. 6.

In step 330, a contour of the ilium region is determined based on the binary image.

In some embodiments, the processing device may process the binary image using a preset screening manner to determine the contour of the ilium region.

The preset screening manner refers to a preset strategy/approach for filtering out the contour of the ilium region from the binary image. In some embodiments, the preset screening manner may include two rounds of screening based on a preset screening strategy. A first round of screening may be used to obtain one or more candidate connected components from the binary image to remove interfering contours, and a second round of screening may be used to obtain the contour of the ilium region based on a result of the first round of screening. Specifically, the processing device may obtain the one or more candidate connected components from the binary image using the preset screening strategy; and determine the contour of the ilium region based on the one or more candidate connected components.

The preset screening strategy is an operational scheme/method for achieving a goal under a specific preset goal and condition. In some embodiments, the preset screening strategy includes screening includes using one or more screening conditions in at least one of three dimensions of area screening, centroid screening, and length screening. For example, the preset screening strategy may be that screening is performed using the centroid screening, or the area screening, or both the centroid screening and the area screening. Preferably, in some embodiments, the screening is performed using the screening condition in three dimensions of the area screening, the centroid screening, and the length screening.

The connected component is a region where a set of pixel points are connected in an image. The candidate connected component is a connected component obtained from the binary image using the preset screening strategy.

The area screening refers to screening a connected component whose area is greater than a certain threshold as a candidate contour according to area. The threshold is related to an area of an image, for example, the bigger the image, the larger the threshold.

The centroid screening refers to screening a connected component whose centroid is in a middle portion of an image. For example, to screen a connected component whose centroid is in the middle one-half of an image.

The length screening refers to screening a connected component whose length is longer than a certain threshold. The threshold of the length screening is related to a width of an image, e.g., the wider the image, the larger the threshold.

In some embodiments, when the count of the one or more candidate connected components is less than 1, the processing device is further configured to obtain a downgraded screening strategy by performing dimensionality reduction of screening conditions in the preset screening strategy, and obtain the one or more candidate connected components from the binary image through a downgraded screening strategy. The dimensionality reduction on the screening conditions refers to discarding one or more screening conditions. In some embodiments, an order of the dimensionality reduction of the screening condition is the length screening, the area screening, and the centroid screening sequentially. For example, the preset screening strategy may be to first screen a connected component using all three of the above screening conditions simultaneously, and when the count of candidate connected components screened is 0 (less than 1), one of the screening conditions is removed, and the screening condition of length screening is removed in order. Screening is performed again through the downgraded screening strategy, and whether the count of candidate connected components screened is less than 1 is determined, and in response to determining the count of candidate connected components screened being still 0, a screening condition is removed again, for example, the screening condition of area screening is removed. After that, the screening is performed again until the count of the one or more candidate connected components is greater than or equal to 1. If the count of the one or more candidate connected components finally is 1, the candidate connected component may be directly output as the ilium region. If the count of the one or more candidate connected components finally is greater than 1, the second round of screening is performed.

In some embodiments, the second round of screening includes determining, for each of the one or more candidate connected components, a discrimination score corresponding to the candidate connected component; designating a candidate connected component, among the one or more candidate connected components, with a highest discrimination score as a target connected component; and extracting the contour of the ilium region from the ultrasound image based on the target connected component. The target connected component is a connected component corresponding to a region where the ilium is located in the ultrasound image.

The discrimination score is a value obtained by calculating the connected component using a preset discrimination algorithm.

In some embodiments, the preset discrimination algorithm may be a discrimination algorithm related to a distance of a connected component, and a formula of the preset discrimination algorithm may be as shown in Equation (7).

$$S = \frac{I}{\max(I_i)_{i=1,N}} + \frac{R}{\max(R_i)_{i=1,N}} + \frac{\theta}{\max(\theta_i)_{i=1,N}} \qquad (7)$$

Where, S denotes a discrimination score corresponding to a connected component, N denotes a count of pixel points in the connected component, i denotes a pixel point, I denotes an average pixel intensity of pixel points in a region corresponding to the connected component in an ultrasound image, R denotes a length-to-width ratio of the connected component, and θ denotes a main axis direction of the connected component.

In calculating the length-to-width ratio R of the connected component, a center distance of the connected component is needed, and a formula for calculating the center distance of the connected component is shown in Equation (8).

$$m_{pq} = \sum_x \sum_y I_{xy}(x - \bar{x})^p (y - \bar{y})^q \qquad (8)$$

Where, $m_{pq}$ denotes a moment of an image, p+q denotes an order, such as second-order moments including $m_{20}, m_{02}, m_{11}$, etc., x and y denote coordinates from the connected component, $\bar{x}$ and $\bar{y}$ denote coordinates of the center of gravity, and $I_{xy}$ denotes a pixel intensity of a pixel point in a region corresponding to the connected component in the ultrasound image, and a formula for calculating the length-to-width ratio R of the connected component is shown in Equation (9).

$$R = 1 - \sqrt{\frac{m_{20}+m_{02}-\sqrt{4m_{11}^2+(m_{20}-m_{02})^2}}{m_{20}+m_{02}+\sqrt{4m_{11}^2+(m_{20}-m_{02})^2}}} \qquad (9)$$

Where $m_{20}$, $m_{02}$, and $m_{11}$ denote second-order moments of an image.

Alternatively, for the main axis direction θ of the connected component, a calculation manner may include the Radon transform, determining based on the second-order central moment, or fitting a line to a set of contour points of the connected component using least squares to calculate the main axis direction.

It should be noted that the above examples are for exemplary purposes only, and the preset discrimination algorithm includes other algorithms, such as an area judgment algorithm, a rectangular box fitting algorithm, a circular fitting algorithm, a pixel density algorithm, etc., which is not limited in the present disclosure.

In some embodiments, the processing device can intercept a portion of a region corresponding to the target connected component from the ultrasound image as a contour of the ilium region corresponding to the hip joint of the subject to be detected.

More descriptions about obtaining the contour of the ilium region can be found in FIG. 6 and the related descriptions thereof.

FIG. 4 is a flowchart illustrating a process for determining an angle of a hip joint according to some embodiments of the present disclosure. In some embodiments, a process 400 may be performed by a processing device (e.g., the processing device 120).

In step 410, a crest point, an end point, and an acetabular labrum midpoint in an ilium region are determined based on an image of a hip joint;

The crest point is typically located at the center position of the caput femoris, which is an approximate position of a rotation center of the hip joint. In some embodiments, the crest point is also referred to as a bone margin turning point.

In some embodiments, the processing device may segment the image of the hip joint into a first image and a second image based on a preset baseline.

The preset baseline is a straight line formed based on a longitudinal coordinate of a centroid of the ilium in the image of the ilium region. For example, the preset baseline may be a straight line that passes through a centroid of the ilium and is parallel with a length direction of image of the ilium region.

The first image is an image of an upper portion of the image of the hip joint after segmenting the image of the hip joint along the preset baseline.

The second image is an image of a lower portion of the image of the hip joint after segmenting the image of the hip joint along the preset baseline.

The processing device may determine an upper borderline of the contour of the ilium in the second image and extract points on the upper borderline to form a set of points of a bone apex line.

The upper borderline is an upper border of the contour of the ilium. The points (e.g., pixel points) on the upper borderline are extracted to obtain the set of points of the bone apex line.

The processing device may obtain the slope of the bone apex line based on the set of points of the bone apex line.

In some embodiments, the processing device may obtain the slope of the bone apex line by performing the least squares line fitting on points on the bone apex line in the set of points of the bone apex line. The slope of the bone apex line may also be obtained by other methods, which are not limited by this embodiment.

The processing device may determine the crest point from the set of points of the bone apex line based on the slope of the bone apex line.

In some embodiments, the processing device may utilize the slope of the bone apex line, combined with the set of points of the bone apex line, to obtain n straight lines (n being a count of the bone apex line points in the set of points of the bone apex line). Longitudinal coordinates of bone apex line points are sorted to ensure that all points in the set of points of the bone apex line are located at the lower-left side of a certain straight line among the n straight lines). Then, coordinates of a point in the set of points of the bone apex line corresponding to the certain straight line are then considered the crest point.

The end point is usually the lowest point of a lower margin of the ilium.

In some embodiments, the processing device may use a point with a maximum longitudinal coordinate in the set of points of the bone apex line as the end point.

The acetabular labrum midpoint is usually a point on the acetabulum.

In some embodiments, the processing device may segment an acetabular labrum region from the first image; and designate a centroid of the acetabular labrum region as the acetabular labrum midpoint.

Segmentation of the acetabular labrum region may be performed in various ways, such as by using the segmentation algorithm described in other embodiments of the present disclosure, which are not limited herein. A method for obtaining the centroid of the acetabular labrum region may be referred to elsewhere in the present disclosure.

In step 420, the bone apex line is determined by connecting the crest point and the end point and a cartilage apex line is determined by connecting the crest point and the acetabular labrum midpoint.

In step 430, an angle of the hip joint of the subject to be detected is determined based on the slope of the bone apex line and the slope of the cartilage apex line.

In some embodiments, the bone apex angle $\alpha$ in the angle of the hip joint may be determined based on the slope of the bone apex line according to Equation (10), and the cartilage apex angle $\beta$ in the angle of the hip joint may be determined based on the slope of the cartilage apex line according to Equation (11).

$$\alpha = \arctan(k1) * \frac{180}{\pi} \qquad (10)$$

$$\beta = \arctan(k2) * \frac{180}{\pi} \qquad (11)$$

where k1 denotes the slope of the bone apex line and k2 denotes the slope of the cartilage apex line.

More descriptions about determining the angle of the hip joint of the subject to be detected can be found in a later description.

Based on the same inventive concept, some embodiments of the present disclosure also provide a method for classifying a hip joint. As shown in FIG. 5, FIG. 5 is a flowchart illustrating a process for classifying a hip joint according to some embodiments of the present disclosure. The method may be applied to a processing device in FIG.1, which may include following steps.

In step 501, an original ultrasound image of a subject to be detected is obtained.

In some embodiments, the original ultrasound image is an ultrasound image corresponding to a hip joint of the subject to be detected, for example, a coronal ultrasound image corresponding to the hip joint. The original ultrasound image may be a colored image or a grayscale image, which is not limited in the present disclosure.

In some embodiments, the processing device may obtain the ultrasound image corresponding to the hip joint of the subject to be detected captured by an ultrasound device from the ultrasound device, or obtain the ultrasound image corresponding to the hip joint of the subject to be detected from a server. It should be understood that the processing device may also obtain ultrasound data corresponding to the hip joint of the subject to be detected acquired by the ultrasound device, and reconstruct the ultrasound image corresponding to the hip joint of the subject to be detected based on original ultrasound data corresponding to the hip joint of the subject to be detected.

In some embodiments, the processing device may also obtain ultrasound image of a part or whole-body of the subject to be detected, and intercept a corresponding ultrasound image of the hip joint from the ultrasound image of the part or whole-body of the subject. It should be noted that a manner for obtaining the ultrasound image of the hip joint of the subject to be detected is not specifically limited in the embodiments of the present disclosure, for example, the processing device may also obtain the ultrasound image of the subject to be detected by reading from a storage device and a database.

In step 502, an image of an ilium region corresponding to the hip joint of the subject to be detected is extracted from the original ultrasound image.

In some embodiments, the processing device may input the ultrasound image corresponding to the hip joint of the subject to be detected into a preset image segmentation model, and obtain the image of the ilium region corresponding to the hip joint of the subject to be detected based on the output of the preset image segmentation model. The preset image segmentation model may be a threshold-based image segmentation model, a region-based image segmentation model, an edge-based image segmentation model, an energy generalization-based image segmentation model (e.g., an active contour model), a deep-learning/neural-network-based image segmentation model, a machine-learning-based image segmentation model, or the like. A type of the preset image segmentation model and a principle of implementation are not specifically limited in this embodiment, as long as the preset image segmentation model is capable of segmenting the image of the ilium region from the ultrasound image of the hip joint.

In some embodiments, the processing device may also perform image analysis on the original ultrasound image using a basic image processing operation, so as to extract the image of the ilium region corresponding to the hip joint of the subject to be detected from the original ultrasound image. The image processing operation includes and is not limited to, image filtering, image smoothing, image geometric transformation, image morphological processing, or the like.

In some embodiments, the processing device may also employ basic image processing operations to analyze the original ultrasound image, so as to extract the image of the ilium region corresponding to the hip joint of the subject to be detected. These image processing operations include, but are not limited to, image filtering, image smoothing, geometric transformations of the image, and morphological image processing.

In some embodiments, extracting the image of the ilium region corresponding to the hip joint of the subject to be detected from the ultrasound image includes filtering the ultrasound image to obtain a filtered ultrasound image, obtaining a binary image including a plurality of contour regions by performing thresholding segmentation on the filtered ultrasound image, determining the contour of the ilium region based on the binary image, and determining the image of the ilium region based on the contour of the ilium region.

In some embodiments, obtaining the image of the ilium region based on the contour of the ilium region includes: obtaining a position of a centroid of the ilium region in the contour of the ilium region, and obtaining the image of the ilium region by expanding in accordance with a preset size based on the position of the centroid of the ilium region in the contour of the ilium region. The preset size correlates with the size of the original ultrasound image.

More specific descriptions can be found in the descriptions of FIG. 2 to FIG. 4, which will not be repeated here.

In step 503, a type of the hip joint of the subject to be detected is obtained by inputting the image of the ilium region into a preset active contour model for hip joint classification.

In some embodiments, the processing device may input the image of the ilium region of the subject to be detected into the preset active contour model to obtain a segmentation image of the hip joint, and then input the segmentation image of the hip joint into a preset classification algorithm, and the preset classification algorithm may output the type of the hip joint of the subject to be detected. The preset active contour model may be trained based on an image sample of an ilium region and a corresponding label of a hip joint. It should be noted that the preset classification algorithm may also be embedded in the preset active contour model, i.e., the preset active contour model may directly output the type of the hip joint of the subject to be detected.

Besides, for the preset classification algorithm, in some embodiments, after obtaining the segmentation image of the hip joint, the processing device may determine a bone apex angle and a cartilage apex angle from the segmentation image of the hip joint. Then, based on a preset correspondence of a type of a hip joint, a type of a hip joint corresponding to the bone apex angle and the cartilage apex angle are determined. The preset correspondence of the type of the hip joint includes correspondences between different bone apex angles, different cartilage apex angles, and different types of hip joints.

In some embodiments, obtaining the type of the hip joint of the subject to be detected by inputting the image of the ilium region into the preset active contour model for hip joint classification includes: processing the image of the ilium region using the preset active contour model to obtaining the image of the hip joint; based on the image of the hip joint, determining a crest point, an end point, and an acetabular labrum midpoint in the ilium region; determining a bone apex line by connecting the crest point and the end point and determining a cartilage apex line by connecting the crest point and the acetabular labrum midpoint; determining the angle of the hip joint of the subject to be detected based on a slope of the bone apex line and a slope of the cartilage apex line; and determining the type of the hip joint of the subject to be detected based on the angle of the hip joint of the subject to be detected and a hip joint classification standard.

In some embodiments, the processing device may determine a classification result of the hip joint of the subject to be detected by looking up the hip joint classification standard.

Exemplarily, the hip joint classification standard may be as shown in Table 1.

Table 1 Hip joint classification standard

| Type of a hip joint | Alpha (α) angle | Beta (β) angle | Classification result (symptom) | Note |
|---|---|---|---|---|
| Ia/Ib | α≥60° | Ia=β<55°Ib=β>55° | Normal Hip | |
| IIa/IIb | α=50-59° | | IIa: physiological immaturity of hip development | IIa <12 weeks |
| | | | IIb: Pathologic immaturity of hip development | IIb> 12 weeks |
| IIc | α=43-49° | β<77° | Severe hip dysplasia | |
| IId | α=43-49° | β>77° | Eccentric hip | |
| III | α<43° | | Hip dislocation | |
| IV | α<43° | | Severe dislocation of the hip joint | |

As shown in Table 1, different bone apex angles α and different cartilage apex angles β correspond to different types of hip joints. By looking up a preset classification template of a hip joint, a type of a hip joint of a subject to be detected corresponding to the bone apex angle and the cartilage apex angle may be determined.

More detailed descriptions can be found in FIG. 2, which will not be repeated here.

In the method for classifying a hip joint, the processing device obtains the original ultrasound image corresponding to the hip joint of the subject to be detected by obtaining the original ultrasound image, then extracts the image of the ilium region corresponding to the hip joint of the subject to be detected from the original ultrasound image, and inputs the image of the ilium region into the preset active contour model for hip joint classification, so as to obtain the type of the hip joint of the subject to be detected. The method for classifying a hip joint provided in the embodiments of the present disclosure obtains the image of the ilium region by segmenting a region where the ilium is located from a complete ultrasound image, and then inputs the image of the ilium region into the active contour model for image analysis, and the active contour model does not need to perform image analysis on the complete image of the hip joint again, only a portion of the image in which a key region is located in the complete hip joint ultrasound image is analyzed, which can greatly improve a processing rate of the active contour model. At the same time, when adopting the technical solution disclosed herein for hip joint classification, the ilium region is extracted from the complete ultrasound image of the hip joint, which means that the region in which the ilium is located is determined from the complete ultrasound image of the hip joint in advance, i.e., a region of interest of the hip joint is determined in advance from the complete ultrasound image of the hip joint. Therefore, when the image of the ilium region is subsequently input to the active contour model for image processing, the accuracy of the segmentation of the hip joint can be greatly improved, and the accuracy of the image processing can be improved.

FIG. 6 is a flowchart illustrating an exemplary process for determining an image of an ilium region according to other embodiments of the present disclosure. The present embodiment relates to an optional implementation of a process for a processing device to extract an image of an ilium region corresponding to a hip joint of a subject to be detected from an original ultrasound image based on the above embodiment, as shown in FIG. 6, step 502 may include:

Step 601, a binary image corresponding to the original ultrasound image is obtained by binarizing the original ultrasound image.

In some embodiments, the processing device adopts simple and fast thresholding segmentation to binarize the original ultrasound image and obtain a corresponding binary image of the original ultrasound image. The thresholding segmentation may include maximum entropy thresholding segmentation, Otsu thresholding segmentation, adaptive thresholding segmentation, fixed thresholding segmentation, etc. Exemplarily, the maximum entropy thresholding segmentation is used to binarize the original ultrasound image, and an obtained binary image can roughly segment the ilium region. In the present disclosure, the obtained binary image by segmentation is also referred to as the image of the ilium region.

The maximum entropy thresholding segmentation utilizes image grayscale probability information to obtain a threshold for binarized segmentation of the original ultrasound image, and then realizes image segmentation. Assuming that the threshold for segmentation of the original ultrasound image is t, pixel points in the original ultrasound image with a gray level less than t constitute a background region B, and pixel points with a gray level greater than or equal to t constitute a target region T, a probability distribution of the individual gray levels is as follows:

$$P_B(i) = \frac{P_i}{P}, i = 0,1,\dots,t \qquad (12)$$

$$P_T(i) = \frac{P_i}{1-P}, i = t + 1, \dots, L - 1 \qquad (13)$$

Where $P_B(i)$ denotes a probability distribution of each pixel point in a background region, $P_T(i)$ denotes a probability distribution of each pixel point in a target region, $P = \sum_{i=0}^{t} P_i$ denotes a probability of a pixel point less than or equal to a segmentation threshold t, $P_i$ denotes a probability of a pixel value of i, L denotes gray levels of the image, and information entropy corresponding to the background and foreground may be expressed as:

$$H(B) = -\sum_{i=0}^{t} P_B(i) ln P_B(i), \ i = 0, 1, \dots, t \qquad (14)$$

$$H(T) = -\sum_{i=t+1}^{L-1} P_T(i) ln P_T(i), i = t + 1, \dots, L - 1 \qquad (15)$$

Where H(B) denotes the information entropy corresponding to the background, H(T) denotes information entropy corresponding to the foreground. A sum of image information entropy is denoted as H(t) = H(T) + H(B). Each of pixel values t from 0 to 255 is traversed as a segmentation threshold, and the total entropy for each threshold is determined. The value t corresponding to a maximum entropy is used as the segmentation threshold, at which point the background and foreground can retain the maximum amount of information. At this point, binarized segmentation of the original ultrasound image can be performed based on the segmentation threshold t, resulting in a segmented image with the maximum entropy, i.e., the binary image corresponding to the original ultrasound image.

Step 602, a target connected component is determined by performing connected component analysis on the binary image.

After performing binarized segmentation on the original ultrasound image, the ilium region can be roughly segmented out, but some interfering contours may still be present, so the connected component analysis can be performed on the binary image corresponding to the original ultrasound image to screen out the target connected component corresponding to the ilium region. In other words, the target connected component is a connected component corresponding to a region where the ilium is located.

In some embodiments, a first preset screening rule for a connected component corresponding to the ilium region may be determined by performing feature analysis on the ilium region. The first preset screening rule may be a screening rule related to at least one of an area of the ilium region, a centroid of the ilium region, a length of the ilium region, or the like. The processing device may perform connected component analysis on the binary image through the first preset screening rule to determine the target connected component. In some embodiments, the processing device may perform the connected component analysis on the binary image to determine all connected components in the binary image, and then, based on that first preset screening rule, determine one or more candidate connected components that satisfy first preset screening rule among all the connected components, and finally, based on the one or more candidate connected components, determine the target connected component corresponding to the ilium region.

In some embodiments, if the count of the one or more candidate connected components is one, the candidate connected component may be used as the target connected component. If the count of the one or more candidate connected components is more than 1, one of the plurality of candidate connected components may be used as the target connected component. If there is an intersection of the plurality of candidate connected components, the plurality of candidate connected components may also be merged and a merged connected component may be used as the target connected component. In some embodiments, the processing device may also screen the target connected component corresponding to the ilium region from the plurality of candidate connected components in accordance with a second preset screening rule. In some embodiments, the second preset screening rule may be a screening rule related to at least one of an area of the connected component, a length-to-width ratio of the connected component, an average pixel intensity in the connected component, or the like.

Step 603, the image of the ilium region corresponding to the hip joint of the subject to be detected is extracted from the original ultrasound image based on the target connected component.

In some embodiments, the processing device may intercept a portion of the original ultrasound image to obtain an image corresponding to the target connected component, and determine the image as the image of the ilium region corresponding to the hip joint of the subject to be detected. In one embodiment, the target connected component may not be a rectangular region, in which case a rectangular region corresponding to the target connected component may be determined based on the target connected component, then the rectangular region is intercepted from the original ultrasound image to obtain the image, and the image is determined as the image of the ilium region corresponding to the hip joint of the subject to be detected.

In some embodiments, the processing device may determine, based on the target connected component, a rectangular region tangent to the target connected component, and the rectangular region encloses the target connected component. The processing device may also first determine a position of a centroid of the target connected component, and then, based on the position of the centroid and a preset size including a preset length and a preset width, determine a rectangular region whose center is located at the position of the centroid and whose size is the same as in the preset size. It should be understandable that other ways may be used, and the present embodiment does not specifically limit how the rectangular region is determined based on the target connected component.

In the present embodiment, the processing device obtains the binary image corresponding to the original ultrasound image by binarizing the original ultrasound image; then, performs the connected component analysis on the binary image to determine the target connected component corresponding to the ilium region; and finally, extracts the image of the ilium region corresponding to the ilium region of the hip joint of the subject to be detected from the original ultrasound image based on the target connected component corresponding to the ilium region. That is, a method for obtaining the image of the ilium region is provided in the present embodiment, which provides an implementable basis for obtaining the image of the ilium region.

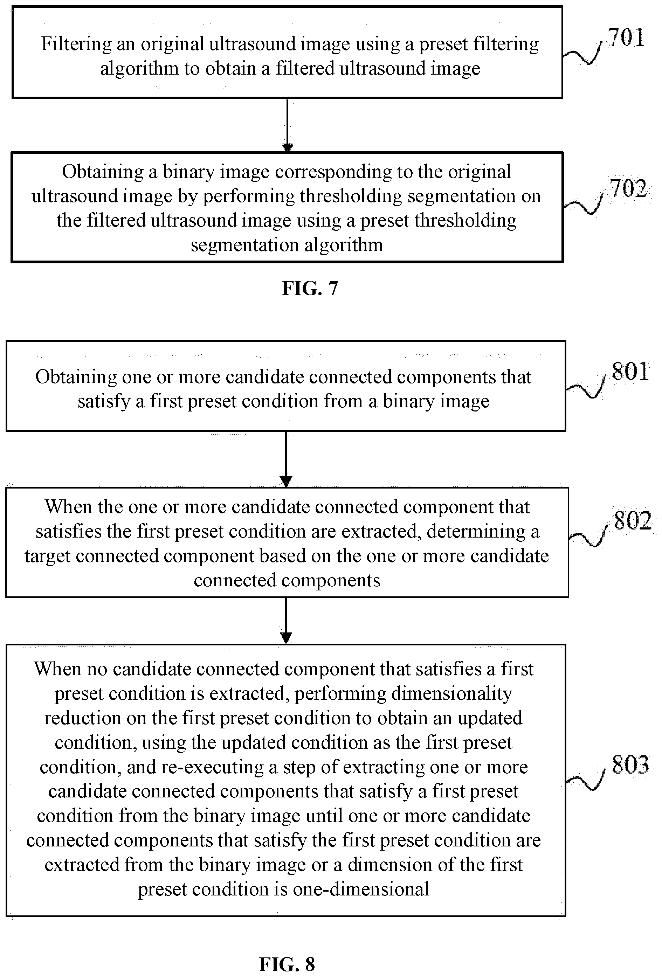

In an optional embodiment of the present disclosure, the processing device, when performing step 601, may also filter the original ultrasound image before binarizing the original ultrasound image to smooth the original ultrasound image, which can suppress speckle noise in the original ultrasound image to some extent, improve the accuracy of the subsequent image segmentation process, and reduce interference during a process of screening a connected component. Based on the above embodiments, as shown in FIG. 7, FIG. 7 is a flowchart illustrating a process for filtering and performing thresholding segmentation on an ultrasound image according to some embodiments of the present disclosure. In some embodiments, step 601 may include:

Step 701, a filtered ultrasound image is obtained by filtering an original ultrasound image using a preset filtering algorithm.

In some embodiments, the preset filtering algorithm may include mean filtering, Gaussian filtering, median filtering, anisotropic diffusion filtering, and so on, which is not limited in the present disclosure.

Step 702, a binary image corresponding to the original ultrasound image is obtained by performing thresholding segmentation on the filtered ultrasound image using a preset thresholding segmentation algorithm.

In some embodiments, the preset thresholding segmentation algorithm may include maximum entropy thresholding segmentation, Otsu thresholding segmentation, adaptive thresholding segmentation, fixed thresholding segmentation, etc., which is not limited in the present disclosure.

For example, the processing device may filter the original ultrasound image using a median screening algorithm to obtain the filtered ultrasound image, and then, perform binary segmentation on the filtered ultrasound image using a maximum entropy thresholding segmentation algorithm to obtain a binary image corresponding to the original ultrasound image.

In the present embodiment, before binarizing the original ultrasound image, the processing device first adopts a preset filtering algorithm to filter the original ultrasound image, obtains the filtered ultrasound image, and then, adopts a preset thresholding segmentation algorithm to perform the binarized segmentation on the filtered ultrasound image to obtain the binary image corresponding to the original ultrasound image. In this way, the noise in the original ultrasound image can be reduced to achieve the effect of smoothing processing, and, in turn, the accuracy of the subsequent image segmentation and the screening of the connected component can be improved to avoid noise interference.

FIG. 8 is a flowchart illustrating a process for obtaining a candidate connected component according to some embodiments of the present disclosure. This embodiment relates to an optional implementation process of a processing device performing connected component analysis on a binary image to determine a target connected component, and based on the above embodiment, as shown in FIG. 8, step 602 may include:

Step 801, one or more candidate connected components that satisfy a first preset condition are obtained from a binary image.

The first preset condition may include a plurality of conditions in a plurality of dimensions, and the plurality of conditions in the plurality of dimensions may include at least two conditions among conditions of an area of a connected component reaching a preset area threshold, a centroid of a connected component being located at a preset position, and a length of a connected component reaching a preset length threshold.

That is to say, the first preset condition includes at least two screening conditions, and in extracting the one or more candidate connected components that satisfy the first preset condition from the binary image, the one or more candidate connected components satisfy both of the at least two screening conditions.

In some embodiments, the processing device may perform connected component analysis on the binary image, determine all connected components in the binary image, and then, for each connected component, determine whether the connected component simultaneously satisfies each condition in the first preset condition. In response to determining that a connected component simultaneously satisfies each condition in the first preset condition, the processing device determines the connected component as the candidate connected component, and in response to determining that the connected component does not satisfy any condition in the first preset condition, the processing device determines that the connected component does not satisfy the screening condition.

Step 802, when the one or more candidate connected components that satisfy the first preset condition are extracted, a target connected component is determined based on the one or more candidate connected components.

That is to say, if one or more candidate connected components satisfy the first preset condition, a target connected component corresponding to an ilium region may be further determined based on the one or more candidate connected components.

In some embodiments, when a count of the one or more candidate connected components is one, the candidate connected component may be used as the target connected component, or a deformed connected component of the candidate connected component (e.g., a rectangular region tangent to the candidate connected component) may be used as the target connected component. When the count of the one or more candidate connected components is more than one, a target connected component that satisfies a second preset condition may be determined from the plurality of connected components, or the target connected component may be determined based on the plurality of candidate connected components. For example, the target connected component is obtained by fusing the plurality of candidate connected components.

In one embodiment, for a scheme for determining the target connected component that satisfies the second preset condition from the plurality of candidate connected components, the second preset condition may be a condition with a highest discrimination score, i.e., discrimination analysis is performed on each of the plurality of candidate connected components to obtain a discrimination score corresponding to each candidate connected component, and a candidate connected component with a highest discrimination score is determined as the target connected component.

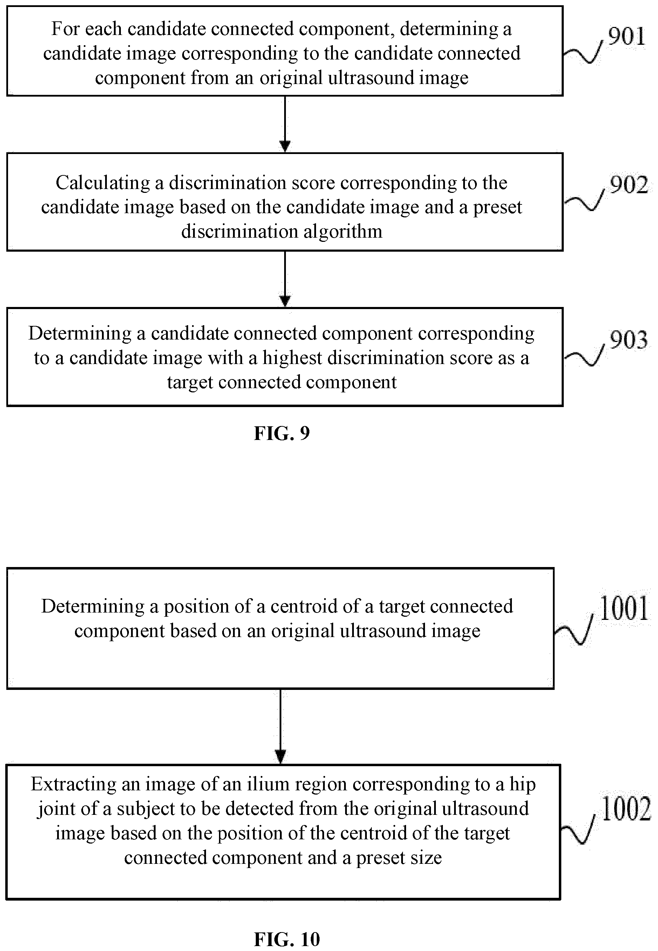

FIG. 9 is a flowchart illustrating a process for determining a target connected component according to some embodiments of the present disclosure. As shown in FIG. 9, a processing device determines a target connected component that satisfies a second preset condition from one or more candidate connected components may include:

Step 901, for each candidate connected component, a candidate image corresponding to the candidate connected component is determined from an original ultrasound image.

Step 902, a discrimination score corresponding to the candidate image is calculated based on the candidate image and a preset discrimination algorithm.

More descriptions regarding step 901 and step 902 can be found in FIG. 3 and the related descriptions thereof.

Step 903, a candidate connected component corresponding to a candidate image with a highest discrimination score is determined as a target connected component.

To this point, the target connected component corresponding to an ilium region may be determined from a plurality of candidate connected components by second screening.

Step 803, when no candidate connected component that satisfies a first preset condition is extracted, an updated condition is obtained by performing dimensionality reduction on the first preset condition, using the updated condition as the first preset condition, and re-executing a step of extracting one or more candidate connected components that satisfy the first preset condition from a binary image until one or more candidate connected components that satisfy the first preset condition are extracted from the binary image or a dimension of the first preset condition is one-dimensional.

That is to say, when screening connected components based on a plurality of screening conditions, if there is no candidate connected component that satisfies the plurality of screening condition, a count of the screening conditions may be reduced and screening of the connected components may be performed anew. In some embodiments, when performing dimensionality reduction on the first preset condition, the dimensionality reduction refers to that a screening condition of low importance may be prioritized to be removed in accordance with an importance degree of each screening condition when reducing the screening conditions of the first preset condition.

For example, when the first preset condition includes an area condition in which an area of a connected component reaches a preset area threshold, a centroid condition in which a position of a centroid of a connected component is located at a preset position, and a length condition in which a length of a connected component reaches a preset length threshold. If there is no candidate connected component that satisfies the first preset condition, the length condition can be removed in priority, and the area condition and the centroid condition may be taken as the first preset condition, and screening of connected components may be performed again. If there is still no candidate connected component that satisfies both the area condition and the centroid condition, then the area condition may be removed, the centroid condition may be taken as the first preset condition and the screening of connected components may be performed again. If there is still no candidate connected component that satisfies the centroid condition, then a step of binarizing an original ultrasound image may be re-executed to re-determine a binary image, and based on the new binary image, screening connected components may be performed in accordance with the screening process until a candidate connected component that satisfies the first preset condition is determined.

Next, after one or more candidate connected components are determined, a target connected component that satisfies the second preset condition may be determined from the one or more candidate connected components with reference to step 802, which will not be repeated herein.

In this embodiment, the processing device first extracts the one or more candidate connected components that satisfy the first preset condition including a plurality of conditions in dimensions from the binary image. When one or more candidate connected components that satisfies the first preset condition are extracted, the target connected component that satisfies the second preset condition is determined from the one or more candidate connected components. When no candidate connected component that satisfies the first preset condition is extracted, the first preset condition is subjected to dimensionality reduction to obtain the updated condition, and the updated condition is used as the first preset condition, and the step of extracting the one or more candidate connected components that satisfy the first preset condition from the binary image is re-executed until the one or more candidate connected components that satisfy the first preset condition is extracted from the binary image or the dimension of the first preset condition is one-dimensional. In this way, the accuracy of segmentation of an ilium region can be improved.

FIG. 10 is a flowchart illustrating a process for determining an image of an ilium region according to some embodiments of the present disclosure. This embodiment relates to an optional implementation process of a processing device for extracting an image of an ilium region corresponding to a hip joint of a subject to be detected from an original ultrasound image based on a target connected component. Based on the above embodiment, as shown in FIG. 10, step 603 may include:

Step 1001, a position of a centroid of a target connected component is determined based on an original ultrasound image.

In some embodiments, a processing device may determine an image region corresponding to the target connected component from the original ultrasound image based on a position of the target connected component in a binary image, perform analysis on the image region corresponding to the target connected component from the original ultrasound image to determine the position of the centroid corresponding to the target connected component.

Step 1002, an image of an ilium region corresponding to a hip joint of a subject to be detected is extracted from the original ultrasound image based on the position of the centroid of the target connected component and a preset size.

The preset size is related to the size of the original ultrasound image. In some embodiments, the processing device may determine a proportional relationship between a size of the image of the ilium region and the size of the ultrasound image by analyzing the ultrasound image and the size of the image of the ilium region in the ultrasound image. For example, the width of the image of the ilium region may be 1/2 of the width of the ultrasound image, and the height of the image of the ilium region may be 4/5 of the width of the ultrasound image.

Based on this, the processing device may determine the preset size corresponding to the image of the ilium region based on the proportional relationship and the size of the original ultrasound image of the subject to be detected. Furthermore, based on the position of the centroid of the target connected component and the preset size, an image region of the preset size centered at the position of the centroid of the target connected component in the original ultrasound image may be determined, and the image region may be intercepted from the original ultrasound image, and the image of the ilium region corresponding to the hip joint of the subject to be detected may be obtained.

In this embodiment, the processing device determines the position of the centroid of the target connected component based on the original ultrasound image, and extracts the image of the ilium region corresponding to the hip joint of the subject to be detected from the original ultrasound image based on the position of the centroid of the target connected component and the preset size, with the preset size being related to the size of the original ultrasound image. In this way, an accurate image of the ilium region may be obtained, and the accuracy of segmentation of the image of the ilium region can be improved.

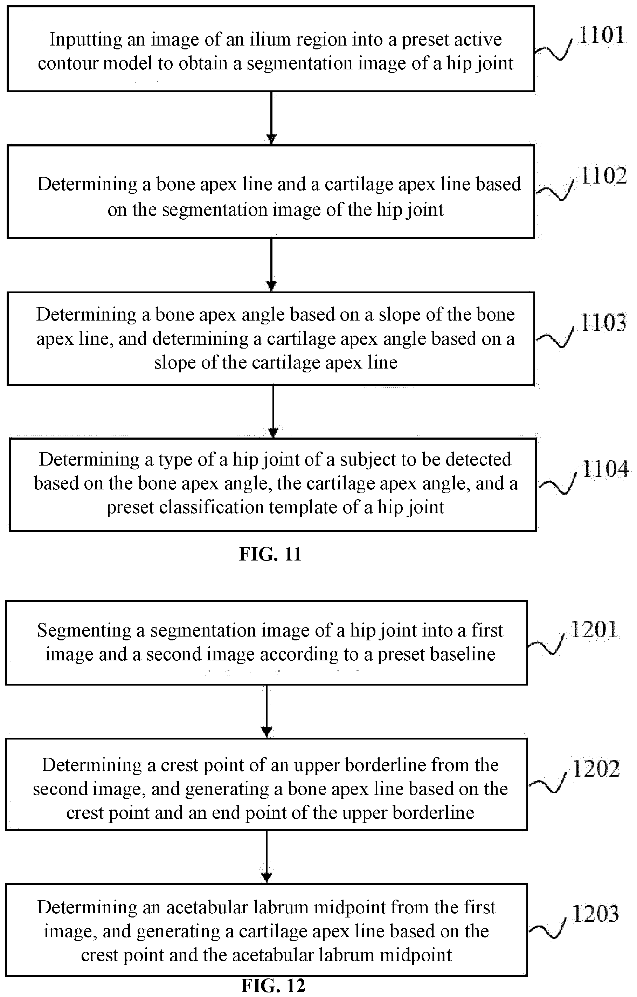

FIG. 11 is a flowchart illustrating a process for classifying a hip joint according to some embodiments of the present disclosure. The present embodiment relates to an optional process performed by a processing device inputting an image of an ilium region into a preset active contour model for hip joint classification to obtain a type of a hip joint of a subject to be detected. Based on the above embodiment, as shown in FIG. 11, step 503 includes:

Step 1101, an image of an ilium region is input into a preset active contour model to obtain a segmentation image of a hip joint.

Step 1102, a bone apex line and a cartilage apex line are determined based on the segmentation image of the hip joint.

The bone apex line is a line between a bone margin turning point and the lowest point of a lower margin of an iliac branch, and the cartilage apex line is a line between the bone margin turning point and an acetabular labrum midpoint.

In some embodiments, a processing device may determine three anatomical points including the bone margin turning point, the lowest point of the lower margin of the iliac branch, and the acetabular labrum midpoint based on the segmentation image of the hip joint. Then, the processing device generates the bone apex line based on the bone margin turning point and the lowest point of the lower margin of the iliac branch, and generates the cartilage apex line based on the bone margin turning point and the acetabular labrum midpoint.

In one embodiment, FIG. 12 is a flowchart illustrating a process for determining three anatomical points according to some embodiments of the present disclosure. As shown in FIG. 12, a process of determining three anatomical points including a bone margin turning point, a lowest point of a lower margin of an iliac branch, and an acetabular labrum midpoint may include:

Step 1201, a segmentation image of a hip joint is segmented into a first image and a second image according to a preset baseline.

The preset baseline is a straight line formed based on a longitudinal coordinate of a centroid of the ilium in an image of an ilium region, and the first image is a portion of the image of the hip joint including the acetabular labrum. The centroid of the ilium in the image of the ilium region is a position of a centroid of a target connected component determined in step 1001, and the straight line formed based on the longitudinal coordinate of the position of the centroid of the target connected component is the preset baseline, that is, a baseline for determining a bone apex angle and a cartilage apex angle of the hip joint.

Based on this preset baseline, the segmentation image of the hip joint may be segmented into the first image corresponding to an upper portion of the hip joint and the second image corresponding to a lower portion of the hip joint.

Step 1202, a crest point of an upper borderline is determined from the second image, and the bone apex line is generated based on the crest point and an end point of the upper borderline.

The crest point of the upper borderline is the bone margin turning point, and the end point of the upper borderline is the lowest point of the lower margin of the iliac branch as described above.

Step 1203, the acetabular labrum midpoint is determined from the first image, and the cartilage apex line is generated based on the crest point and the acetabular labrum midpoint.

In some embodiments, the processing device may perform morphological processing and logical filtering on the first image to segment an image of a region where the acetabular labrum is located, and based on the image, determine a centroid of the region where the acetabular labrum is located, and determine the centroid as the acetabular labrum midpoint.

After the acetabular labrum midpoint is determined, the cartilage apex line is generated based on the crest point determined in step 1202 and the acetabular labrum midpoint.

Step 1103, the bone apex angle is determined based on a slope of the bone apex line, and the cartilage apex angle is determined based on a slope of the cartilage apex line.

The bone apex angle is an angle formed by the preset baseline and the bone apex line in the fourth quadrant, and the cartilage apex angle is an angle formed by the preset baseline and the cartilage apex line in the first quadrant.

After determining the bone apex line, the slope of the bone apex line may be calculated by coordinate computation, and similarly, after determining the cartilage apex line, the slope of the cartilage apex line may be calculated. Then, a manner of calculating the bone apex angle and the cartilage apex angle based on the slope of the bone apex line and the slope of the cartilage apex line can be found in the description of the previous embodiment, which will not be repeated herein.

Step 1104, a type of the hip joint of the subject to be detected is determined based on the bone apex angle, the cartilage apex angle, and a preset classification template of hip joint.

The preset classification template of a hip joint can be seen in Table 1.

In the present embodiment, the processing device obtains the segmentation image of the hip joint by inputting the image of the ilium region into the preset active contour model; next, based on the segmentation image of the hip joint, determines the bone apex line and the cartilage apex line; determines the bone apex angle based on the slope of the bone apex line, determines the cartilage apex angle based on the slope of the cartilage apex line; and then, determines the type of the hip joint of the subject to be detected based on the bone apex angle, the cartilage apex angle, and the preset classification template for a hip joint. By adopting the method in the present embodiment, using a region of interest of the hip joint, i.e., the image of the ilium region, as an input image of segmentation of the hip joint, a computation time can be shortened, a segmentation rate and the accuracy of the segmentation can be increased, which can improve the accuracy and processing efficiency of segmentation of the hip joint.

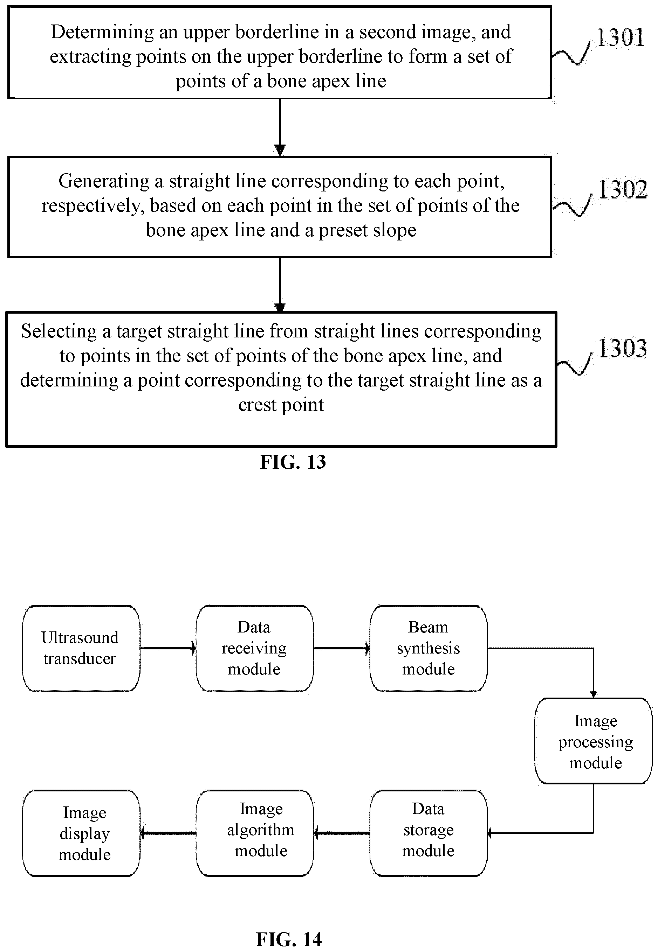

FIG. 13 is a flowchart illustrating a process for determining a crest point according to some embodiments of the present disclosure. This embodiment relates to an optional implementation of a process performed by a processing device to determine a crest point of an upper borderline from a second image. Based on the above embodiment and as shown in FIG. 13, step 1202 includes:

Step 1301, an upper borderline is determined in a second image, and points on the upper borderline are extracted to form a set of points of a bone apex line.

The upper borderline in the second image is an upper boundary line corresponding to a lower margin of an iliac branch.

In some embodiments, a plurality of points between a first point and a last point on the upper borderline are extracted at a preset interval to form the set of points of the bone apex line.

Step 1302, a straight line corresponding to each point is generated, respectively, based on each point in the set of points of the bone apex line and a preset slope. The straight line corresponding to each point has a slope same as the preset slope.

The preset slope is a slope of a line segment obtained by fitting the points in the set of points of the bone apex line using the least squares method. In other words, by using the least squares method to fit the set of points of the bone apex line, a fitted line and its slope are obtained, and the slope of the line is used as the preset slope.

Based on this, the preset slope is utilized in conjunction with the set of points of the bone apex line to obtain the straight line that corresponds to each point in the set of points of the bone apex line, respectively.

In step 1303, a target straight line is selected from straight lines corresponding to the points in the set of points of the bone apex line and a point corresponding to the target straight line is determined as the crest point.

Each of the points in the set of points of the bone apex line is below the target straight line.

In some embodiments, a processing device may sort longitudinal coordinates of the points in the set of points of the bone apex line to ensure that all the points in the set of points of the bone apex line are all located at a left and lower part of the target straight line, and then a point corresponding to the target straight line in the set of points of the bone apex line is the crest point, and a point with a maximum longitudinal coordinate in the set of points of the bone apex line is regarded as an end point of the upper borderline, and then a bone apex line is obtained by connecting the crest point and the end point, and the slope of the bone apex line is calculated.

In this embodiment, the processing device determines the upper borderline in the second image, determining the set of points of the bone apex line by extracting the points on the upper borderline, generates straight lines corresponding to points in the set of points, based on each point in the set of points of the bone apex line and the preset slope; then, the processing device selects the target straight line from the straight lines, and determines the point corresponding to the target straight line as the crest point. Each point in the set of points of the bone apex line is below the target straight line, and the slope of the line segment is obtained by fitting the points in the set of points of the bone apex line using the least squares method. In the present embodiment, a method for determining a crest point is provided, which improves the feasibility and operability of a processing device in automatically determining the crest point, and the method can improve the processing efficiency of the processing device in determining the crest point as well as improve the accuracy of the crest point.

In some embodiments, a system for hip joint automatic classification is also provided. A structure of the system is as shown in FIG. 14, which is divided into seven main parts. An ultrasound transducer is mainly configured to transmit and receive a signal, a data receiving module is mainly configured to receive, amplify, and perform analog-to-digital conversion on an electrical signal, compresses data, and send the data to a beam synthesis module, the beam synthesis module and an image processing module are mainly configured to parse and interpolate an echo signal to form a B signal, and a data storage module is configured to store the acquired B signal in a form of image, and send a stored original ultrasound image to an image algorithm module to automatically obtain the hip joint classification, and finally an image display module is configured to display a classification result.

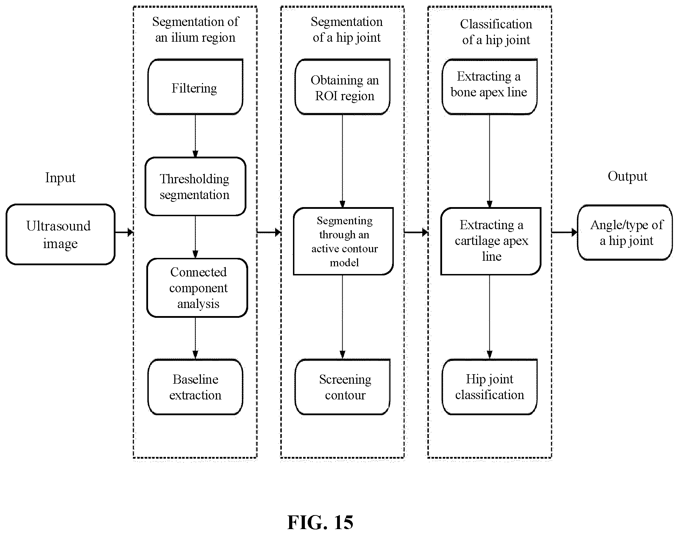

The image algorithm module is specifically configured to perform steps of a method for classifying a hip joint in any of the above embodiments to realize hip joint automatic classification. A manner for realizing the method may be as shown in FIG. 15, and an entire algorithmic process is divided into five parts: input, segmentation of an ilium region, segmentation of a hip joint, classification of a hip joint, and output. The method for classifying a hip joint in the embodiment of the present disclosure is a fully automated measurement method, where a doctor selects an appropriate section of a hip joint of a subject to be detected as an input image in a frozen situation, and a final output is an alpha angle, a beta angle, and a hip joint classification of the hip joint of the subject to be detected. A specific process for realization may include following steps including:

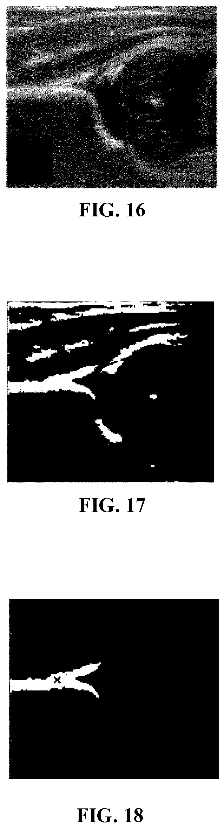

(1) obtaining an original ultrasound image corresponding to the hip joint of the subject to be detected, as shown in FIG. 16.

(2) filtering the original ultrasound image using a median filtering algorithm to obtain a filtered ultrasound image; then, performing binarized segmentation on the filtered ultrasound image using a maximum entropy thresholding segmentation algorithm to obtain a binary image corresponding to the original ultrasound image, as shown in FIG. 17.

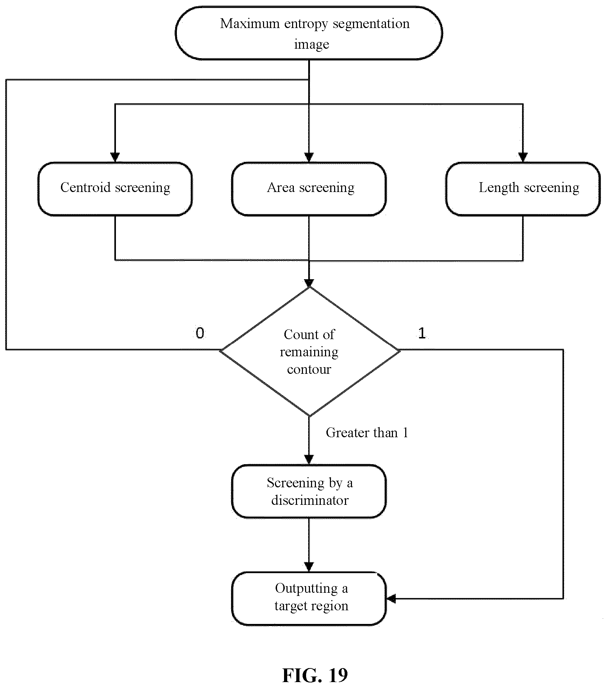

(3) performing connected component analysis on the binary image to determine a target connected component where an ilium region is located, as shown in FIG. 18. Referring to FIG.19, a specific process for realization may include:

a. extracting one or more candidate connected components that satisfy a first preset condition from the binary image (i.e., a maximum entropy segmentation image in FIG. 19); where the first preset condition includes an area condition in which an area of a connected component reaches a preset area threshold, a centroid condition in which a centroid of a connected component is located at a preset position, and a length condition in which a length of a connected component reaches a preset length threshold.

b. when no candidate connected component that satisfies the first preset condition is extracted (i.e., a count of remaining contours is 0), performing dimensionality reduction on the first preset condition to obtain an updated condition, and using the updated condition as the first preset condition and re-execute step 1) until one or more candidate connected components that satisfy the first preset condition are extracted from the binary image or a dimension of the first preset condition is one-dimensional. An order of the dimensionality reduction is to remove the length condition first and then the area condition.

c. when the one or more candidate connected components that satisfy the first preset condition are extracted, if a count of the one or more candidate connected components is one, designating the one candidate connected component as the target connected component.

d. If the count of the one or more candidate connected components is more than one, then for each candidate connected component, determining a candidate image corresponding to each candidate connected component from the original ultrasound image, and inputting each candidate image into a preset discriminator to calculate a discrimination score corresponding to each candidate image, and designating a candidate connected component corresponding to a candidate image with a highest discrimination score as the target connected component.

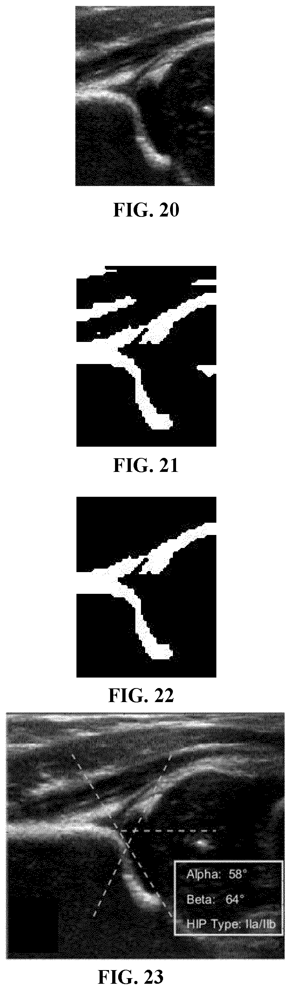

(4) determining a position of a centroid of the target connected component based on the original ultrasound image, such as a position of a centroid labeled in FIG. 18; and extracting an image of the ilium region corresponding to the hip joint of the subject to be detected from the original ultrasound image based on the position of the centroid of the target connected component and a preset size, as shown in FIG. 20; where the preset size is correlated with a size of the original ultrasound image.

(5) Inputting the ilium region image into a preset active contour model to obtain a segmentation image of the hip joint, as shown in FIG. 21; then, screening a contour of the hip joint from the segmentation image of the hip joint to obtain a segmentation image of the hip joint that includes only the contour of the hip joint, as shown in FIG. 22.

(6) determining a preset baseline, a bone apex line, and a cartilage apex line based on the segmentation image of the hip joint that includes only the contour of the hip joint. A specific process for realization may include:

a. segmenting the segmentation image of the hip joint into a first image and a second image according to a preset baseline; the preset baseline is a straight line formed based on a longitudinal coordinate of a centroid of the ilium in the image of the ilium region, and the first image is a portion of the image of the hip joint image including the acetabular labrum.

b. for the second image, determining an upper borderline in the second image, extracting points on the upper borderline to form a set of points of the bone apex line; and generating a straight line corresponding to each point according to each point in the set of points of the bone apex line and a preset slope; next, selecting a target straight line from the straight lines corresponding to the points, and determining a point corresponding to the target straight line as a crest point; and designating a point with a maximum value of a vertical coordinate in the set of points of the bone apex line as an end point; finally, generating the bone apex line by connecting the crest point and the end point.

c. segmenting a region where the acetabular labrum is located from the first image and determining a centroid of the region where the acetabular labrum is located, and determining the centroid as an acetabular labrum midpoint, and generating the cartilage apex line by connecting the crest point and the acetabular labrum midpoint.

(7) determining a bone apex angle based on a slope of the bone apex line and determining a cartilage apex angle based on a slope of the cartilage apex line.

(8) determining the type of the hip joint of the subject to be detected based on the bone apex angle, the cartilage apex angle, and a preset classification template for a hip joint.

As shown in FIG. 23, where Alpha denotes an $\alpha$ angle, Beta denotes a $\beta$ angle, and HIP Type denotes a type of a hip joint.

In embodiments of the present disclosure, the method for classifying a hip joint obtains a segmentation result of a hip joint by first obtaining the segmentation image of the ilium region and extracting the centroid of the segmentation image of the ilium region, and then obtaining a region of interest of the hip joint with the position of the centroid as a benchmark, and then inputting the region of interest of the hip joint into the active contour model for further detailed segmentation. Compared to inputting the whole ultrasound image into the active contour model, this process can enhance a processing speed and processing accuracy of an active contour model algorithm, and thus improve the accuracy of the measurement of an angle of the hip joint, as well as improve the speed and accuracy of segmentation of the hip joint.

In addition, the method for classifying a hip joint provided in this embodiment only requires inputting the original ultrasound image of the subject to be detected to obtain a classification result of a hip joint, which significantly improves the diagnostic efficiency for doctors and reduces the waiting time for patients. In addition, the fully automated method ensures that the classification result is more objective, reducing misdiagnosis caused by the lack of experience of some doctors. Moreover, as compared to a deep learning method, the method provided by embodiments of the present disclosure does not need to rely on a large amount of data for training, and only needs to rely on a small number of ultrasound images for algorithm validation, which greatly saves the cost of the algorithm.

Further, the system for hip joint ultrasound automatic classification provided in this embodiment includes the entire process from receiving the signal through the ultrasonic transducer, converting the signal into a B-mode image via beamforming, data storage, image algorithm processing, and result display, which enhances the system's integrity and reliability.

It should be appreciated that although the individual steps in the flowcharts involved in the embodiments as described above are shown sequentially as indicated by the arrows, the steps are not necessarily executed sequentially in the order indicated by the arrows. Unless expressly stated herein, there is no strict order limitation on the execution of these steps, and the steps may be executed in some other order. Moreover, at least some of the steps in the flowcharts of the embodiments described above may include multiple steps or stages. These steps or stages do not necessarily have to be completed at the same time; rather, they can be executed at different times. The execution order of these steps or stages does not have to follow a sequential order and may be performed alternately or in parallel with other steps or stages, or parts of those steps or stages, from other steps.

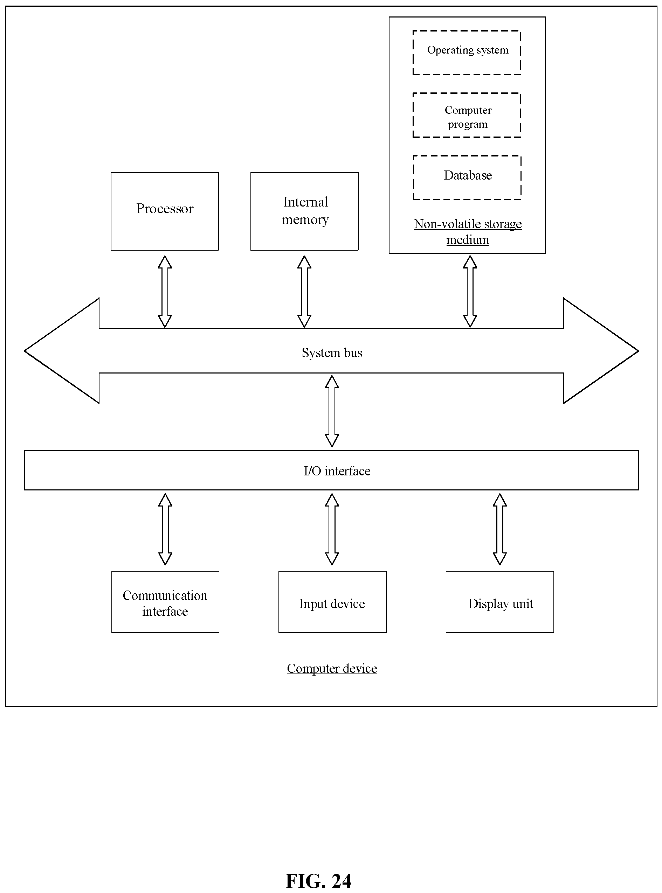

FIG. 24 is a schematic diagram illustrating an internal structure of a processing device according to some embodiments of the present disclosure.

In some embodiments, a computer device (which may also be referred to as a processing device, e.g., the processing device 120) is provided, which may be an ultrasound device, a server communicatively connected to the ultrasound device, a terminal device communicatively connected to the ultrasound device, and a terminal device communicatively connected to the server, etc. when the computer device is the terminal device, an internal structure of the computer device may be shown in FIG. 24. The computer device includes a processor, a memory, a communication interface, a display unit, and an input device connected via a system bus. The communication interface, the display unit, and the input device may be connected to the system bus via an I/O (input/output) interface. The processor of the computer device is configured to provide computing and control capabilities. The memory of the computer device includes a non-volatile storage medium and an internal memory. The non-volatile storage medium stores an operating system and a computer program. The internal memory provides an environment for operation of the operating system and the computer program in the non-volatile storage medium. The communication interface of the computing device is configured to communicate with external terminals in wired or wireless mode, which can be realized through Wi-Fi, mobile cellular networks, NFC (Near Field Communication), or other technologies. The computer program is executed by the processor to execute a method for classifying a hip joint. A display screen of the computer device may be an LCD or an e-ink display, and the input device of the computer device may be a touch layer overlaid on the display screen or a key provided on a shell of the computer device, a trackball, or a touchpad, and may be an external keyboard, touchpad, or mouse.

It will be understood by those skilled in the art that a structure shown in FIG. 24 is merely a block diagram of part of the structure related to the solution presented in the present disclosure, and does not constitute a limitation on the computer device to which the solution is applied. A specific computer device may include more or fewer components than those shown in the diagram, or may combine certain components, or may have a different component arrangement. It is to be understood that the system and its modules shown in FIG. 24 may be implemented utilizing a variety of means. For example, in some embodiments, the system and its modules may be implemented by hardware, software, or a combination of software and hardware. In this regard, the hardware portion may be implemented utilizing dedicated logic; the software portion may be stored in memory and executed by an appropriate instruction execution system, such as a microprocessor or dedicated design hardware. Those skilled in the art will appreciate that the methods and systems described above may be implemented using computer-executable instructions and/or included in processor control code, such as provided on carrier media such as disks, CDs or DVD-ROMs, programmable memory such as read-only memories (firmware), or data carriers such as optical or electronic signal carriers. The system and its modules of the present disclosure may be realized not only with hardware circuits such as ultra-large scale integrated circuits or gate arrays, semiconductors such as logic chips, transistors, etc., or programmable hardware devices such as field-programmable gate arrays, programmable logic devices, etc., but also with software executed, for example, by processors of various types, or with a combination of the above hardware circuits and software (e.g., firmware).

It should be noted that the description of the system for classifying a hip joint and its modules above is for convenience of explanation only and should not be construed as limiting the scope of the present disclosure. It can be understood that, for those skilled in the art, after understanding the principles of the system, the various modules may be combined in any manner without departing from these principles, or form subsystems connected to other modules. For example, the modules may share a common storage module, or each module may have its own separate storage module. Such variations are all within the protection scope of the present disclosure.

The basic concepts have been described above, and it is apparent to those skilled in the art that the foregoing detailed disclosure serves only as an example and does not constitute a limitation of the present disclosure. While not expressly stated herein, a person skilled in the art may make various modifications, improvements, and amendments to the present disclosure. Those types of modifications, improvements, and amendments are suggested in the present disclosure, so those types of modifications, improvements, and amendments remain within the spirit and scope of the exemplary embodiments of the present disclosure.

Also, the present disclosure uses specific words to describe embodiments of the present disclosure, such as "an embodiment", "one embodiment", and/or "some embodiment" means a feature, structure, or characteristic associated with at least one embodiment of the present disclosure. Accordingly, it should be emphasized and noted that "one embodiment" or "an embodiment" or "an alternative embodiment" in different places in the present disclosure do not necessarily refer to the same embodiment. In addition, certain features, structures, or characteristics in one or more embodiments of the present disclosure may be suitably combined.

In addition, unless explicitly stated in the claims, the order of the processing elements and sequences, the use of numerical or alphabetical designations, or the use of other names in the present disclosure are not intended to limit the order of the processes and methods described herein. Although some of the embodiments of the invention that are currently considered useful have been discussed through various examples in the disclosure above, it should be understood that such details are for illustrative purposes only. The appended claims are not limited to the disclosed embodiments; rather, the claims are intended to cover all modifications and equivalent combinations that fall within the spirit and scope of the embodiments disclosed herein. For example, while the system components described above may be implemented through hardware devices, they could also be implemented solely through software solutions, such as by installing the described system on existing servers or mobile devices.

Similarly, it should be noted that, in order to simplify the presentation of the disclosure in the present disclosure and to aid in understanding one or more embodiments of the invention, multiple features may sometimes be combined into a single embodiment, figure, or description thereof in the earlier parts of the present disclosure. However, this disclosure method does not imply that the features required by the subject of the present disclosure are more than those mentioned in the claims. In fact, the features of an embodiment are fewer than all the features disclosed in the single embodiment described above.

Some embodiments use numbers to describe the number of components, attributes, and it should be understood that such numbers used in the description of embodiments are modified in some examples by the modifiers "approximately", "nearly", or "substantially". Unless otherwise noted, the terms "approximately", "nearly", or "substantially" indicates that a $\pm20\%$ variation in the stated number is allowed. Correspondingly, in some embodiments, the numerical parameters used in the present disclosure and claims are approximations, which can change depending on the desired characteristics of individual embodiments. In some embodiments, the numerical parameters should consider the specified number of valid digits and employ general place-keeping. While the numerical domains and parameters used to confirm the breadth of their ranges in some embodiments of the present disclosure are approximations, in specific embodiments such values are set to be as precise as possible within a feasible range.

For each of the patents, patent applications, patent application disclosures, and other materials cited in the present disclosure, such as articles, books, specification sheets, publications, documents, etc., the entire contents of which are hereby incorporated herein by reference. Application history documents that are inconsistent with or conflict with the contents of the present disclosure are excluded, as are documents (currently or hereafter appended to the present disclosure) that limit the broadest scope of the claims of the present disclosure. It should be noted that in the event of any inconsistency or conflict between the descriptions, definitions, and/or use of terms in the materials appended to the present disclosure and those set forth herein, the descriptions, definitions, and/or use of terms in the present disclosure shall prevail.

Finally, it should be understood that the embodiments described in the present disclosure are only used to illustrate the principles of the embodiments of the present disclosure. Other deformations may also fall within the scope of the present disclosure. As such, alternative configurations of embodiments of the present disclosure may be considered to be consistent with the teachings of the present disclosure as an example, not as a limitation. Correspondingly, the embodiments of the present disclosure are not limited to the embodiments expressly presented and described herein. lie in less than all features of a single foregoing disclosed embodiment.

**Claims**

1.   A system for detecting an angle of a hip joint, the system comprising a processor, wherein the processor is configured to:

   obtain an ultrasound image of a subject to be detected;
   extract a contour of an ilium region corresponding to a hip joint of the subject to be detected from the ultrasound image;
   obtain a position of a centroid of the ilium region in the contour of the ilium region;
   obtain an image of the hip joint based on the position of the centroid; and
   determine an angle of the hip joint of the subject to be detected based on the image of the hip joint.

2.   The system of claim 1, wherein the extracting a contour of an ilium region corresponding to a hip joint of the subject to be detected from the ultrasound image includes:

   filtering the ultrasound image to obtain a filtered ultrasound image;
   obtaining a binary image including a plurality of contour regions by performing thresholding segmentation on the filtered ultrasound image; and
   determining the contour of the ilium region based on the binary image.

**3.** The system of claim 2, wherein a filtering manner includes median filtering, and a thresholding segmentation manner includes maximum entropy thresholding segmentation.

**4.** The system of claim 2, wherein the determining the contour of the ilium region based on the binary image includes: determining the contour of the ilium region by processing the binary image using a preset screening manner.

**5.** The system of claim 4, wherein the processing the binary image using a preset screening manner includes:

obtaining one or more candidate connected components from the binary image through a preset screening strategy, wherein the preset screening strategy includes using at least one of screening conditions in three dimensions of area screening, centroid screening, and length screening; and
determining the contour of the ilium region based on the one or more candidate connected components.

**6.** The system of claim 5, wherein when a count of the one or more candidate connected components is less than 1, the processor is further configured to:

obtain a downgraded screening strategy by performing dimensionality reduction on the screening conditions of the preset screening strategy; and
obtain one or more candidate connected components from the binary image through the downgraded screening strategy.

**7.** The system of claim 6, wherein an order of the dimensionality reduction on the screening conditions is the length screening, the area screening, and the centroid screening sequentially.

**8.** The system of claim 5, wherein when the count of the one or more candidate connected components is greater than 1, the processor is further configured to:

calculate, for each of the one or more candidate connected components, a discrimination score corresponding to the candidate connected component;
designate a candidate connected component, among the one or more candidate connected components, with a highest discrimination score as a target connected component; and
extract the contour of the ilium region from the ultrasound image based on the target connected component.

**9.** The system of claim 1, wherein the obtaining an image of the hip joint based on the position of the centroid includes:

obtaining an image of the ilium region by expanding in accordance with a preset size based on the position of the centroid of the ilium region in the contour of the ilium region, wherein the preset size correlates with a size of the ultrasound image; and
obtaining the image of the hip joint by processing the image of the ilium region using a preset active contour model.

**10.** The system of claim 9, wherein the determining an angle of the hip joint of the subject to be detected based on the image of the hip joint includes:

determining a crest point, an end point, and an acetabular labrum midpoint of the ilium region based on the image of the hip joint;
determining a bone apex line by connecting the crest point and the end point;
determining a cartilage apex line by connecting the crest point and the acetabular labrum midpoint; and
determining the angle of the hip joint of the subject to be detected based on a slope of the bone apex line and a slope of the cartilage apex line.

**11.** The system of claim 10, wherein the determining the crest point based on the image of the hip joint includes:

segmenting the image of the hip joint into a first image and a second image according to a preset baseline, the preset baseline being a straight line formed based on a longitudinal coordinate of a centroid of an ilium in the image of the ilium region;
determining an upper borderline of a contour of the ilium in the second image;
extracting points on the upper borderline to form a set of points of the bone apex line;
obtaining the slope of the bone apex line based on the set of points of the bone apex line; and

determining the crest point from the set of points of the bone apex line based on the slope of the bone apex line.

12. The system of claim 11, wherein the determining the end point based on the image of the hip joint includes:
designating a point corresponding to a maximum longitudinal coordinate in the set of points of the bone apex line as the end point.

13. The system of claim 11, wherein the determining the acetabular labrum midpoint based on the image of the hip joint includes:

segmenting an acetabular labrum region from the first image; and
designating a centroid of the acetabular labrum region as the acetabular labrum midpoint.

14. A method for detecting an angle of a hip joint, the method comprising:

obtaining an ultrasound image of a subject to be detected;
extracting a contour of an ilium region corresponding to a hip joint of the subject to be detected from the ultrasound image;
obtaining a position of a centroid of the ilium region in the contour of the ilium region;
obtaining an image of the hip joint based on the position of the centroid; and
determining an angle of the hip joint of the subject to be detected based on the image of the hip joint.

15. The method of claim 14, wherein the extracting a contour of an ilium region corresponding to a hip joint of the subject to be detected from the ultrasound image includes:

filtering the ultrasound image to obtain a filtered ultrasound image;
obtaining a binary image including a plurality of contour regions by performing thresholding segmentation on the filtered ultrasound image; and
determining the contour of the ilium region based on the binary image.

16. The method of claim 15, wherein a filtering manner includes median filtering, and a thresholding segmentation manner includes maximum entropy thresholding segmentation.

17. The method of claim 15, wherein the determining the contour of the ilium region based on the binary image includes:
determining the contour of the ilium region by processing the binary image using a preset screening manner.

18. The method of claim 17, wherein the processing the binary image using a preset screening manner includes:

obtaining one or more candidate connected components from the binary image through a preset screening strategy, wherein the preset filtering strategy includes using at least one of screening conditions in three dimensions of area screening, centroid screening, and length screening; and
determining the contour of the ilium region based on the one or more candidate connected components.

19. The method of claim 18, wherein when a count of the one or more candidate connected components is less than 1, the processor is further configured to:

obtain a downgraded screening strategy by performing dimensionality reduction on the screening conditions of the preset screening manner; and
obtain the one or more candidate connected components from the binary image through the downgraded screening strategy.

20. The method of claim 19, wherein an order of the dimensionality reduction on the screening conditions is the length screening, the area screening, and the centroid screening sequentially.

21. The method of claim 18, wherein when the count of the one or more candidate connected components is greater than 1, the processor is further configured to:

calculate, for each of the one or more candidate connected components, a discrimination score corresponding to the candidate connected component;

designate a candidate connected component, among the one or more candidate connected components, with a highest discrimination score as a target connected component; and

extract the contour of the ilium region from the ultrasound image based on the target connected component.

22. The method of claim 14, wherein the obtaining an image of the hip joint based on the position of the centroid includes:

obtaining an image of the ilium region by expanding in accordance with a preset size based on the position of the centroid of the ilium region in the contour of the ilium region, wherein the preset size correlates with a size of the ultrasound image; and

obtaining the image of the hip joint by processing the image of the ilium region using a preset active contour model.

23. The method of claim 22, wherein the determining an angle of the hip joint of the subject to be detected based on the image of the hip joint includes:

determining a crest point, an end point, and an acetabular labrum midpoint of the ilium region based on the image of the hip joint;

determining a bone apex line by connecting the crest point and the end point;

determining a cartilage apex line by connecting the crest point and the acetabular labrum midpoint; and

determining the angle of the hip joint of the subject to be detected based on a slope of the bone apex line and a slope of the cartilage apex line.

24. The method of claim 23, wherein the determining the crest point based on the image of the hip joint includes:

segmenting the image of the hip joint into a first image and a second image according to a preset baseline; the preset baseline being a straight line formed based on a longitudinal coordinate of a centroid of an ilium in the image of the ilium region;

determining an upper borderline of a contour of the ilium in the second image;

extracting points on the upper borderline to form a set of points of the bone apex line;

obtaining the slope of the bone apex line based on the set of points of the bone apex line; and

determining the crest point from the set of points of the bone apex line based on the slope of the bone apex line.

25. The method of claim 24, wherein the determining the end point based on the image of the hip joint includes:
designating a point corresponding to a maximum longitudinal coordinate in the set of points of the bone apex line as the end point.

26. The method of claim 24, wherein the determining the acetabular labrum midpoint based on the image of the hip joint includes:

segmenting an acetabular labrum region from the first image; and

designating a centroid of the acetabular labrum region as the acetabular labrum midpoint.

27. A method for hip joint classification, the method comprising:

obtaining an original ultrasound image of a subject to be detected, the original ultrasound image being an ultrasound image corresponding to a hip joint of the subject to be detected;

extracting an image of an ilium region corresponding to the hip joint of the subject to be detected from the original ultrasound image; and

obtaining a type of the hip joint of the subject to be detected by inputting the image of the ilium region into a preset active contour model for hip joint classification.

28. The method of claim 27, wherein the extracting an image of an ilium region corresponding to the hip joint of the subject to be detected from the original ultrasound image includes:

filtering the original ultrasound image to obtain a filtered original image;

obtaining a binary image including a plurality of contour regions by performing thresholding segmentation on the filtered original ultrasound image;

determining a contour of the ilium region based on the binary image; and

determining the image of the ilium region based on the contour of the ilium region.

29. The method of claim 28, wherein the determining the contour of the ilium region based on the binary image includes: determining the contour of the ilium region by processing the binary image through a preset screening manner.

30. The method of claim 29, wherein the processing the binary image through a preset screening manner includes:

obtaining one or more candidate connected components from the binary image through a preset screening strategy, wherein the preset screening strategy includes using at least one of screening conditions in three dimensions of area screening, centroid screening, and length screening; and
determining the contour of the ilium region based on the one or more candidate connected components.

31. The method of claim 28, wherein the determining the image of the ilium region based on the contour of the ilium region includes:

obtaining a position of a centroid of the ilium region in the contour of the ilium region; and
obtaining the image of the ilium region by expanding in accordance with a preset size based on the position of the centroid of the ilium region in the contour of the ilium region, wherein the preset size correlates with a size of the ultrasound image.

32. The method of claim 27, wherein the obtaining the type of the hip joint of the subject to be detected by inputting the image of the ilium region into a preset active contour model for classification of a hip joint includes:

obtaining the image of the hip joint by processing the image of the ilium region using the preset active contour model;
determining a crest point, an end point, and an acetabular labrum midpoint of the ilium region based on the image of the hip joint;
determining a bone apex line by connecting the crest point and the end point, and determining a cartilage apex line by connecting the crest point and the acetabular labrum midpoint;
determining the angle of the hip joint of the subject to be detected based on a slope of the bone apex line and a slope of the cartilage apex line; and
determining the type of the hip joint of the subject to be detected based on the angle of the hip joint of the subject to be detected and a hip joint classification standard.

33. A device for classifying a hip joint, the device comprising a processor, wherein the processor is configured to execute the method for hip joint classification of any one of claims 27 to 32.

100

FIG. 1

200

Obtaining an ultrasound image of a subject to be detected ~/210

Extracting a contour of an ilium region corresponding to a hip joint of the subject to be detected from the ultrasound image ~/220

Obtaining a position of a centroid of the ilium region in the contour of the ilium region and obtaining an image of the hip joint based on the position of the centroid ~/230

Determining an angle of the hip joint of the subject to be detected based on the image of the hip joint ~/240

**FIG. 2**

300

| Filtering an ultrasound image to obtain a filtered ultrasound image | 310 |

↓

| Obtaining a binary image including a plurality of contour regions by performing thresholding segmentation on the filtered ultrasound image | 320 |

↓

| Determining a contour of an ilium region based on the binary image | 330 |

**FIG. 3**

400

| Determining a crest point, an end point, and an acetabular labrum midpoint in an ilium region based on an image of a hip joint | 410 |

↓

| Determining a bone apex line by connecting the crest point and the end point and determining a cartilage apex line by connecting the crest point and the acetabular labrum midpoint | 420 |

↓

| Determining an angle of a hip joint of a subject to be detected based on a slope of the bone apex line and a slope of the cartilage apex line | 430 |

**FIG. 4**

Obtaining an original ultrasound image of a subject to be detected  ⟋501

Extracting an image of an ilium region corresponding to a hip joint of the subject to be detected from the original ultrasound image  ⟋502

Obtaining a type of the hip joint of the subject to be detected by inputting the image of the ilium region into a preset active contour model for hip joint classification  ⟋503

**FIG. 5**

Binarizing an original ultrasound image to obtain a binary image corresponding to the original ultrasound image  ⟋601

Performing connected component analysis on the binary image to determine a target connected component  ⟋602

Extracting an image of an ilium region corresponding to a hip joint of a subject to be detected from the original ultrasound image based on the target connected component  ⟋603

**FIG. 6**

Filtering an original ultrasound image using a preset filtering algorithm to obtain a filtered ultrasound image

~/ 701

Obtaining a binary image corresponding to the original ultrasound image by performing thresholding segmentation on the filtered ultrasound image using a preset thresholding segmentation algorithm

~/ 702

**FIG. 7**

Obtaining one or more candidate connected components that satisfy a first preset condition from a binary image

~/ 801

When the one or more candidate connected component that satisfies the first preset condition are extracted, determining a target connected component based on the one or more candidate connected components

~/ 802

When no candidate connected component that satisfies a first preset condition is extracted, performing dimensionality reduction on the first preset condition to obtain an updated condition, using the updated condition as the first preset condition, and re-executing a step of extracting one or more candidate connected components that satisfy a first preset condition from the binary image until one or more candidate connected components that satisfy the first preset condition are extracted from the binary image or a dimension of the first preset condition is one-dimensional

~/ 803

**FIG. 8**

For each candidate connected component, determining a candidate image corresponding to the candidate connected component from an original ultrasound image  ∿901

Calculating a discrimination score corresponding to the candidate image based on the candidate image and a preset discrimination algorithm  ∿902

Determining a candidate connected component corresponding to a candidate image with a highest discrimination score as a target connected component  ∿903

**FIG. 9**

Determining a position of a centroid of a target connected component based on an original ultrasound image  ∿1001

Extracting an image of an ilium region corresponding to a hip joint of a subject to be detected from the original ultrasound image based on the position of the centroid of the target connected component and a preset size  ∿1002

**FIG. 10**

Inputting an image of an ilium region into a preset active contour model to obtain a segmentation image of a hip joint ⟋1101

Determining a bone apex line and a cartilage apex line based on the segmentation image of the hip joint ⟋1102

Determining a bone apex angle based on a slope of the bone apex line, and determining a cartilage apex angle based on a slope of the cartilage apex line ⟋1103

Determining a type of a hip joint of a subject to be detected based on the bone apex angle, the cartilage apex angle, and a preset classification template of a hip joint ⟋1104

**FIG. 11**

Segmenting a segmentation image of a hip joint into a first image and a second image according to a preset baseline ⟋1201

Determining a crest point of an upper borderline from the second image, and generating a bone apex line based on the crest point and an end point of the upper borderline ⟋1202

Determining an acetabular labrum midpoint from the first image, and generating a cartilage apex line based on the crest point and the acetabular labrum midpoint ⟋1203

**FIG. 12**

EP 4 557 235 A1

Determining an upper borderline in a second image, and extracting points on the upper borderline to form a set of points of a bone apex line    1301

Generating a straight line corresponding to each point, respectively, based on each point in the set of points of the bone apex line and a preset slope    1302

Selecting a target straight line from straight lines corresponding to points in the set of points of the bone apex line, and determining a point corresponding to the target straight line as a crest point    1303

FIG. 13

Ultrasound transducer → Data receiving module → Beam synthesis module → Image processing module

Image display module ← Image algorithm module ← Data storage module ← Image processing module

FIG. 14

36

**FIG. 15**

FIG. 16

FIG. 17

FIG. 18

**FIG. 19**

**FIG. 20**

**FIG. 21**

**FIG. 22**

**FIG. 23**

Operating system

Computer
program

Database

Non-volatile storage
medium

Processor

Internal
memory

System bus

I/O interface

Communication
interface

Input device

Display unit

Computer device

**FIG. 24**

<div align="center">

**INTERNATIONAL SEARCH REPORT**

</div>

| International application No. |
| --- |
| **PCT/CN2023/121265** |

**A. CLASSIFICATION OF SUBJECT MATTER**

G06V 10/764(2022.01)i

According to International Patent Classification (IPC) or to both national classification and IPC

**B. FIELDS SEARCHED**

Minimum documentation searched (classification system followed by classification symbols)

IPC: G06V, G06K, G06N

Documentation searched other than minimum documentation to the extent that such documents are included in the fields searched

Electronic data base consulted during the international search (name of data base and, where practicable, search terms used)

DWPI, CNABS, WPABS, CNTXT, ENTXT, CJFD, CNKI: 髋关节, 髋部, 关节, 超声, 图像, 髂骨, 质心, 角度, 骨缘, 骨顶角, 髋臼, 区域, 轮廓, 滤波, 二值, 灰度, 归一, 阈值分割, 连通域, 面积, hip, joint, ultrasound, image, ilium, centroid, angle, limbus, apex angle, acetabulum, region, contour, filtering, binary, grayscale, normalization, thresholding, connectivity, area

**C. DOCUMENTS CONSIDERED TO BE RELEVANT**

| Category* | Citation of document, with indication, where appropriate, of the relevant passages | Relevant to claim No. |
| --- | --- | --- |
| PX | CN 115527065 A (WUHAN UNITED IMAGING MEDICAL TECHNOLOGY CO., LTD.) 27 December 2022 (2022-12-27) description, paragraphs 75-199 | 1-33 |
| X | CN 109919943 A (GUANGDONG WOMEN AND CHILDREN HOSPITAL) 21 June 2019 (2019-06-21) description, paragraphs 3-77 | 1-26 |
| Y | CN 109919943 A (GUANGDONG WOMEN AND CHILDREN HOSPITAL) 21 June 2019 (2019-06-21) description, paragraphs 3-77 | 27-33 |
| Y | CN 107146214 A (XIAMEN UNIVERSITY; YICHUANG (XIAMEN) MEDICAL TECHNOLOGY CO., LTD.) 08 September 2017 (2017-09-08) description, paragraphs 8-20 | 27-33 |
| A | CN 108537838 A (BEIJING INSTITUTE OF TECHNOLOGY) 14 September 2018 (2018-09-14) entire document | 1-33 |

☑ Further documents are listed in the continuation of Box C.      ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **11 January 2024** | **11 January 2024** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

International application No.

**PCT/CN2023/121265**

### C. DOCUMENTS CONSIDERED TO BE RELEVANT

| Category* | Citation of document, with indication, where appropriate, of the relevant passages | Relevant to claim No. |
|---|---|---|
| A | US 2021209399 A1 (DONDERA, R.; WISNIOWSKI, M.) 08 July 2021 (2021-07-08) entire document | 1-33 |

Form PCT/ISA/210 (second sheet) (July 2022)

**INTERNATIONAL SEARCH REPORT**
Information on patent family members

International application No.

**PCT/CN2023/121265**

| Patent document cited in search report | | | Publication date (day/month/year) | Patent family member(s) | | | Publication date (day/month/year) |
|---|---|---|---|---|---|---|---|
| CN | 115527065 | A | 27 December 2022 | None | | | |
| CN | 109919943 | A | 21 June 2019 | CN | 109919943 | B | 10 November 2020 |
| CN | 107146214 | A | 08 September 2017 | None | | | |
| CN | 108537838 | A | 14 September 2018 | CN | 108537838 | B | 01 December 2020 |
| US | 2021209399 | A1 | 08 July 2021 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202211180701